(19) Europäisches Patentamt
European Patent Office
Office européen des brevets

(11) Veröffentlichungsnummer: **0 078 989**
**B1**

(12) **EUROPÄISCHE PATENTSCHRIFT**

(45) Veröffentlichungstag der Patentschrift: **08.05.85**

(51) Int. Cl.⁴: **C 07 D 237/20**, C 07 D 237/22, A 61 K 31/50

(21) Anmeldenummer: **82109956.1**

(22) Anmeldetag: **28.10.82**

# (54) Pyridazinonimine und ihre physiologisch verträglichen Säureadditionssalze, Verfahren zu ihrer Herstellung und diese Verbindungen enthaltende therapeutische Mittel.

(30) Priorität: **06.11.81 DE 3144138**

(43) Veröffentlichungstag der Anmeldung: **18.05.83 Patentblatt 83/20**

(45) Bekanntmachung des Hinweises auf die Patenterteilung: **08.05.85 Patentblatt 85/19**

(84) Benannte Vertragsstaaten: **AT BE CH DE FR GB IT LI NL SE**

(56) Entgegenhaltungen:
**DE - A - 1 542 693**
**FR - A - 2 150 745**

(73) Patentinhaber: **BASF Aktiengesellschaft, Carl-Bosch-Strasse 38, D-6700 Ludwigshafen (DE)**

(72) Erfinder: **Kropp, Rudolf, Sprottauer Strasse 2, D-6703 Limburgerhof (DE)**
Erfinder: **Thyes, Marco, Dr., Thorwaldsenstrasse 1, D-6700 Ludwigshafen (DE)**
Erfinder: **Schlecker, Rainer, Dr., Suedring 8, D-6719 Bissersheim (DE)**
Erfinder: **Franke, Albrecht, Dr., Mandelring 11, D-6706 Wachenheim (DE)**
Erfinder: **Reicheneder, Franz, Dr., Wingerstrasse 22, D-6700 Ludwigshafen (DE)**
Erfinder: **Amann, August, Dr., Parkstrasse 30, D-6700 Ludwigshafen (DE)**
Erfinder: **Teschendorf, Hans-Juergen, Dr., Rene-Bohn-Strasse 4, D-6700 Ludwigshafen (DE)**
Erfinder: **Kretzschmar, Rolf, Dr., Oberer Bergelweg 4, D-6718 Gruenstadt (DE)**
Erfinder: **Traut, Martin, Dr., Muehltalstrasse 125, D-6900 Heidelberg (DE)**
Erfinder: **Gries, Josef, Dr., Roemerweg 43, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft neue, am Iminstickstoffatom substituierte, 1-Phenyl-4(1H)-pyridazinonimine der Formel (I)

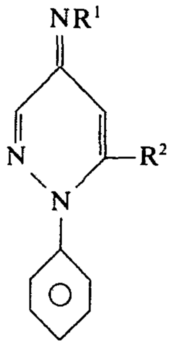

(I)

und ihre Säureadditionssalze mit einer physiologisch verträglichen Säure, Verfahren zu ihrer Herstellung, diese Verbindungen enthaltende pharmazeutische Zubereitungen welche als Arzneimittel, insbesondere als Antidepressiva, Antiparkinsonmittel und Antihypotonika eingesetzt werden können.

Es wird darauf hingewiesen, daß die Säureadditionssalze der Verbindungen der Formel (I) auch als Pyridaziniumsalze gemäß Formel (II) formuliert

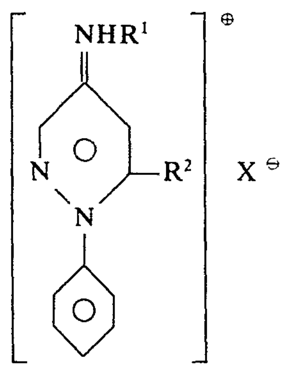

(II)

werden können, wobei $X^{\ominus}$ das Säureanion bedeutet.

Es ist bereits eine Reihe von verschiedenartig substituierten 4(1H)-Pyridazinoniminen beschrieben worden, beispielsweise in der BE-C-645 360 oder in den deutschen Offenlegungsschriften 1 542 693 und 1 770 772, für die eine Anwendung als Zwischenprodukte für die Herstellung von Farbstoffen, Insektiziden und Pflanzenschutzmitteln oder als Herbizid angegeben wird. Aus den deutschen Offenlegungsschriften 1 912 941, 2 139 687, 2 211 662 und 2 245 248 sind Pyridazininiumsalze mit einem Aminrest in 4-Stellung hervor, die als Zwischenprodukte für die Herstellung von Farbstoffen, Pharmazeutika und Schädlingsbekämpfungsmitteln und teilweise als Arzneimittel mit antidepressiver und blutdrucksteigernder Wirkung beschrieben werden.

Die in spezieller Weise substituierten Verbindungen, wie sie Gegenstand der vorliegenden Erfindung sind, sind noch nicht hergestellt worden.

Es wurde nun gefunden, daß 1-Phenyl-4(1H)-pyridazinonimine der Formel (I),

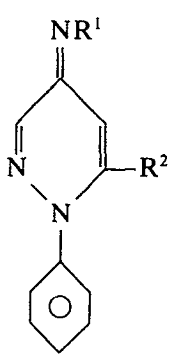

(I)

in der $R^1$ eine der folgenden Bedeutungen hat:

- einen Acylrest $-CO-R^3$, in dem $R^3$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, einen Cycloalkylrest mit 3 bis 8 C-Atomen im Ring, der gegebenenfalls ein- bis vierfach durch Alkylreste mit 1 bis 4 C-Atomen substituiert ist, einen Alkenylrest mit 2 bis 8 C-Atomen oder einen Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, steht,
- einen Rest $-CO-Y-R^4$, in dem Y für ein Sauerstoffatom oder ein Schwefelatom und $R^4$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, einen Alkenylrest mit 3 bis 8 C-Atomen, in dem die Doppelbindung durch eine Alkylenkette mit mindestens 1 C-Atom von Y getrennt ist, oder einen Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, stehen,
- einen Rest

$$-\overset{\overset{\displaystyle Y}{\|}}{C}-NH-R^5$$

in dem Y für ein Sauerstoff- oder Schwefelatom und $R^5$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, oder einen Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, stehen, oder einen Rest $-CO-COO-R^6$, in dem $R^6$ für einen Alkylrest mit 1 bis 8 C-Atomen steht, und

$R^2$ ein Wasserstoffatom oder einen Alkoxyrest mit 1 bis 8 C-Atomen im Alkyl bedeuten,

und ihre Säureadditionssalze mit einer physiologisch verträglichen anorganischen oder organischen Wasserstoffsäure wertvolle pharmakologische Eigenschaften aufweisen.

Für die genannten Reste seien beispielsweise genannt:

Die Alkylreste mit 1 bis 8 C-Atomen können für den Rest $R^3$ in der Acylgruppe $-CO-R^3$, den Rest $R^4$ in der Gruppe $-CO-Y-R^4$, den Rest $R^5$ in der Gruppe

$$-\overset{\overset{\displaystyle Y}{\|}}{C}-NH-R^5$$

oder den Rest $R^6$ in der Gruppe $-CO-COO-R^6$, geradkettig oder verzweigt sein. Zum Beispiel Methyl, Ethyl, Propyl, Isopropyl, Butyl, Isobutyl, sec-Butyl, tert-Butyl, Pentyl, 1-Methylbutyl, 2-Methylbutyl, Isopentyl, 1,1-Dimethylpropyl, 2,2-Dimethylpropyl, Hexyl, Heptyl und Octyl.

Die Substituenten des ein- bis dreifach durch gleiche oder verschiedene Reste substituierten Phenylrings sind Alkyl mit 1 bis 3 C-Atomen, wie Methyl, Ethyl oder Propyl, Alkoxy mit 1 bis 3 C-Atomen im Alkyl, wie Methoxy oder Ethoxy, Halogen, wie Chlor, Brom oder Fluor, oder Trifluormethyl. Die substituierte Benzylgruppe, die für den Rest $R^3$ in der Acylgruppe $-CO-R^3$, den Rest $R^4$ in der Gruppe $-CO-Y-R^4$ oder den Rest $R^5$ in der Gruppe

$$-\overset{\overset{\displaystyle Y}{\|}}{C}-NH-R^5$$

steht, kann z. B. sein 2-Methylbenzyl, 3-Methylbenzyl, 4-Methylbenzyl, 4-Ethylbenzyl, 2,4-Dimethylbenzyl, 2,5-Dimethylbenzyl, 3,4-Dimethylbenzyl, 3,5-Dimethylbenzyl, 2-Methoxybenzyl, 3-Methoxybenzyl, 4-Methoxybenzyl, 4-Ethoxybenzyl, 2,3-Dimethoxybenzyl, 2,4-Dimethoxybenzyl, 2,5-Dimethoxybenzyl, 3,4-Dimethoxybenzyl, 3,5-Dimethoxybenzyl, 3,4,5-Trimethoxybenzyl, 2-Chlorbenzyl, 3-Chlorbenzyl, 4-Chlorbenzyl, 2,4-Dichlorbenzyl, 2,5-Dichlorbenzyl, 2,6-Dichlorbenzyl, 3,4-Dichlorbenzyl, 3,5-Dichlorbenzyl, 2-Brombenzyl, 3-Brombenzyl, 4-Brombenzyl, 2-Fluorbenzyl, 3-Fluorbenzyl, 4-Fluorbenzyl, 2-Chlor-4-fluorbenzyl, 2-Chlor-6-fluorbenzyl, 4-Chlor-2-fluorbenzyl, 5-Chlor-2-fluorbenzyl, 2-(Trifluormethyl)benzyl, 3-(Trifluormethyl)benzyl und 4-(Trifluormethyl)benzyl;

als Cycloalkylreste mit 3 bis 8 C-Atomen im Ring, die unsubstituiert oder ein- bis vierfach durch Alkylreste mit 1 bis 4 C-Atomen substituiert sein können und für den Rest $R^3$ in der Acylgruppe $-CO-R^3$ stehen, kommen beispielsweise in Frage: Cyclopropyl, 1-Methylcyclopropyl, 2-Methylcyclopropyl,

2,2-Dimethylcyclopropyl, 1,2,2-Trimethylcyclopropyl, 2,2,3-Trimethylcyclopropyl, 2,2,3,3-Tetramethylcyclopropyl, 1-Butylcyclopropyl, Cyclobutyl, 1-Methylcyclobutyl, 2-Methylcyclobutyl, 3-Methylcyclobutyl, 3,3-Dimethylcyclobutyl, 1-Propylcyclobutyl, 1-Butylcyclobutyl, 3-Tertiärbutylcyclobutyl, Cyclopentyl, 1-Methylcyclopentyl, 2,5-Dimethylcyclopentyl, Cyclohexyl, 1-Methylcyclohexyl, 2-Ethylcyclohexyl, Cycloheptyl und Cyclooctyl;
Alkenylreste mit 2 bis 8 C-Atomen die den Rest $R^3$ in der Acylgruppe $-CO-R^3$ definieren sind z. B.: Vinyl, Prop-1-enyl, Isopropenyl, Allyl, 1-Methylprop-1-enyl, 2-Methylprop-1-enyl, 1-Methylprop-2-enyl, But-1-enyl, But-2-enyl, But-3-enyl, 1,2-Dimethylprop-1-enyl, 1-Ethylprop-1-enyl, 2-Methylbut-1-enyl, 3-Methylbut-1-enyl, 1-Methylbut-2-enyl, 2-Methylbut-2-enyl, Pent-1-enyl, Pent-2-enyl, Pent-3-enyl, Pent-4-enyl, 1-Ethylbut-1-enyl, 2-Ethylbut-1-enyl, 1,3-Dimethylbut-1-enyl, 2-Methylpent-1-enyl, 3-Methylpent-1-enyl, 3-Methylpent-2-enyl, 4-Methylpent-3-enyl, Hex-3-enyl, Hex-5-enyl, 2-Ethyl-1-methylbut-1-enyl, 1,4-Dimethylpent-3-enyl, Hept-1-enyl, Hept-6-enyl, Oct-1-enyl und Oct-7-enyl.

Als Alkenylreste mit 3 bis 8 C-Atomen die den Rest $R^4$ in der Gruppe $-CO-Y-R^4$ definieren, wobei die Doppelbindung durch eine Alkylenkette mit mindestens 1 C-Atom von Y getrennt ist, sind beispielsweise: Allyl, But-2-enyl, But-3-enyl, 1-Methylallyl, 2-Methylallyl, 1,1-Dimethylallyl, 1-Ethylallyl, 1-Methylbut-2-enyl, 3-Methylbut-2-enyl, 1-Methylbut-3-enyl, 3-Methylbut-3-enyl, Pent-4-enyl, 1-Propylallyl, Hex-2-enyl, Hex-3-enyl, 1,5-Dimethylhex-4-enyl und 1-Pentylallyl.

Substituierte Phenylreste, die ein- bis dreifach durch gleiche oder verschiedene Substituenten aus der Gruppe Alkyl mit 1 bis 4 C-Atomen, wie Methyl, Ethyl oder Propyl, Alkoxy mit 1 bis 4 C-Atomen im Alkyl, wie Methoxy oder Ethoxy, Halogen, wie Chlor, Brom oder Fluor, oder Trifluormethyl substituiert sein können und für den Rest $R^3$ in der Acylgruppe $-CO-R^3$, den Rest $R^4$ in der Gruppe $-CO-Y-R^4$ und den Rest $R^5$ in der Gruppe

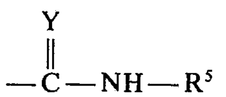

stehen sind z. B.: 2-Tolyl, 3-Tolyl, 4-Tolyl, 2-Ethylphenyl, 3-Ethylphenyl, 4-Ethylphenyl, 2-Propylphenyl, 4-Propylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2,4,6-Trimethylphenyl, 2,3,5-Trimethylphenyl, 2,3,6-Trimethylphenyl, 2-Methoxyphenyl, 3-Methoxyphenyl, 4-Methoxyphenyl, 2-Ethoxyphenyl, 4-Ethoxyphenyl, 2,3-Dimethoxyphenyl, 2,4-Dimethoxyphenyl, 2,5-Dimethoxyphenyl, 2,6-Dimethoxyphenyl, 3,4-Dimethoxyphenyl, 3,5-Dimethoxyphenyl, 2,3,4-Trimethoxyphenyl, 2,4,5-Trimethoxyphenyl, 2,4,6-Trimethoxyphenyl, 3,4,5-Trimethoxyphenyl, 2-Chlorphenyl, 3-Chlorphenyl, 4-Chlorphenyl, 2-Bromphenyl, 3-Bromphenyl, 4-Bromphenyl, 2-Fluorphenyl, 3-Fluorphenyl, 4-Fluorphenyl, 2,3-Dichlorphenyl, 2,4-Dichlorphenyl, 2,5-Dichlorphenyl, 2,6-Dichlorphenyl, 3,4-Dichlorphenyl, 3,5-Dichlorphenyl, 2,4-Difluorphenyl, 2,5-Difluorphenyl, 2,6-Difluorphenyl, 2-Chlor-6-fluorphenyl, 3-Chlor-4-fluorphenyl, 2-(Trifluormethyl)phenyl, 3-(trifluormethyl)phenyl, 4-(Trifluormethyl)phenyl, 2-Methoxy-5-methylphenyl, 3-Methoxy-4-methylphenyl, 4-Methoxy-2-methylphenyl, 4-Methoxy-3-methylphenyl, 2-Ethoxy-4-ethylphenyl, 2-Chlor-4-methylphenyl, 2-Chlor-5-methylphenyl, 2-Chlor-6-methylphenyl, 3-Chlor-2-methylphenyl, 3-Chlor-4-methylphenyl, 4-Chlor-2-methylphenyl, 4-Chlor-3-methylphenyl, 5-Chlor-2-methylphenyl, 2-Chlor-5-methoxyphenyl, 3-Chlor-4-methoxyphenyl, 3-Chlor-5-methoxyphenyl, 4-Chlor-2-methoxyphenyl, 5-Chlor-2-methoxyphenyl, 4-Chlor-2,5-dimethoxyphenyl, 5-Chlor-2,4-dimethoxyphenyl, 3-Fluor-4-methylphenyl, 5-Fluor-2-methylphenyl, 3-Fluor-4-methoxyphenyl, 5-Methoxy-3-(trifluormethyl)phenyl, 2-Chlor-5-(trifluormethyl)phenyl, 4-Chlor-2-(trifluormethyl)phenyl, 4-Chlor-3-(trifluormethyl)phenyl, 4-Fluor-2-(trifluormethyl)phenyl und 4-Fluor-3-(trifluormethyl)phenyl.

$R^2$ kann einen Alkoxyrest mit 1 bis 8 C-Atomen im Alkyl bedeuten wie z. B. Methoxy, Ethoxy, Propoxy, Isopropoxy, Butoxy, sec-Butoxy, Isobutoxy, tert-Butoxy, Pentyloxy, 1-Methylbutoxy, 2-Methylbutoxy, Isopentyloxy, 1,1-Dimethylpropoxy, 2,2-Dimethylpropoxy, Hexyloxy, Heptyloxy und Octyloxy.

Von den genannten Bedeutungen sind Verbindungen der Formel (I) bevorzugt, in der

$R^1$ einen Acylrest $-CO-R^3$, in dem $R^3$ für einen Alkylrest mit 1 bis 8 C-Atomen, einen Benzylrest, der im Phenylring einfach durch Fluor, Chlor oder Methoxy substituiert sein kann, einen Cycloalkylrest mit 3 bis 6 C-Atomen im Ring, der gegebenenfalls ein- oder zweifach durch eine Methylgruppe substituiert ist, einen Alkenylrest mit 2 bis 4 C-Atomen oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, steht,
einen Rest $-CO-Y-R^4$, in dem Y für ein Sauerstoffatom oder ein Schwefelatom und $R^4$ für einen Alkylrest mit 1 bis 4 C-Atomen, einen Benzylrest, der im Phenylring einfach durch Fluor, Chlor oder Methoxy substituiert sein kann, einen Alkenylrest mit 3 bis 6 C-Atomen, in dem die Doppelbindung durch eine Alkylenkette mit mindestens 1 C-Atom von Y getrennt ist, oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, stehen,

einen Rest

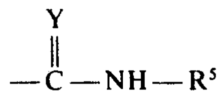

in dem Y für ein Sauerstoff- oder Schwefelatom und $R^5$ für einen Alkylrest mit 1 bis 6 C-Atomen, einen Benzylrest, der im Phenylring einfach durch Fluor, Chlor oder Methoxy substituiert sein kann, oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, stehen, oder

einen Rest $-CO-COO-R^6$, in dem $R^6$ für einen Alkylrest mit 1 bis 4 C-Atomen steht, und

$R^2$ ein Wasserstoffatom oder einen Alkoxyrest mit 1 bis 4 C-Atomen im Alkyl bedeuten,

und ihre Säureadditionssalze mit einer physiologisch verträglichen Säure.

Als physiologisch verträgliche Säuren kommen die üblichen physiologisch verträglichen anorganischen oder organischen Wasserstoffsäuren, wie z. B. Chlor- oder Bromwasserstoffsäure, Phosphorsäure, Schwefelsäure, Methylschwefelsäure, Ethylschwefelsäure, Oxalsäure, Maleinsäure, Fumarsäure, Milchsäure, Weinsäure, Apfelsäure, Zitronensäure, Salicylsäure, Adipinsäure oder Benzoesäure, in Betracht.

Übliche physiologisch verträgliche Säuren können auch aus Fortschritte der Arzneimittelforschung, Band 10, Seiten 224 und 225, Birkhäuser Verlag, Basel und Stuttgart, 1966 oder dem Journal of Pharmaceutical Sciences, Volume 66, Seiten 1 bis 5 (1977) entnommen werden.

Die erfindungsgemäßen Pyridazinonimine der Formel (I), in denen $R^1$ für eine Acylgruppe $-CO-R^3$ steht, und ihre Säureadditionssalze mit einer physiologisch verträglichen Säure können hergestellt werden, indem man eine Verbindung der Formel (III)

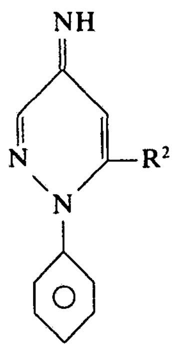

(III)

in der $R^2$ die für die Formel (I) angegebenen Bedeutungen hat, gegebenenfalls in Form eines beliebigen Säureadditionssalzes, mit einem Acylierungsmittel der Formel (IV)

$$R^3—CO—A \qquad (IV)$$

in der A für ein Halogenatom, insbesondere ein Chloratom, oder den Rest $-O-CO-R^3$ steht, wobei $R^3$ obige Bedeutung hat, steht, in an sich bekannter Weise umsetzt und die erhaltene Verbindung, wenn es sich um das freie Imin handelt, ggf. in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

Die Umwandlung eines bei dem Herstellungsverfahren erhaltenen Säureadditionssalzes kommt vor allem dann in Betracht, wenn eine Ausgangsverbindung der Formel (III) in Form eines Säureadditionssalzes verwendet wird, das nicht unbedingt als physiologisch verträglich bezeichnet werden kann.

Gemäß den für A angegebenen Bedeutungen sind zweckmäßige Acylierungsmittel die entsprechenden Carbonsäurehalogenide, insbesondere Chloride, und die entsprechenden Carbonsäureanhydride.

Die Acylierung einer freien Verbindung der Formel (III) mit einem Acylierungsmittel der Formel (IV) wird unter an sich üblichen Bedingungen durchgeführt. In der Regel unter Verwendung von wenigstens einer äquimolaren Menge des Acylierungsmittels, zweckmäßig in Gegenwart eines Lösungs- oder Verdünnungsmittels, gegebenenfalls in einem Zwei-Phasensystem, bestehend aus Wasser und einem mit Wasser nicht mischbaren Lösungsmittel, gegebenenfalls in Gegenwart einer Base als säurebindendes Mittel und gegebenenfalls unter Druck.

Verbindungen der Formel (III), in denen $R^2$ für einen Alkoxyrest steht, werden zweckmäßigerweise bei Temperaturen von 0 bis 50°C umgesetzt, Verbindungen, in denen $R^2$ für ein Wasserstoffatom steht, bei Temperaturen von 0 bis 120°C, vorzugsweise 0 bis 80°C. Innerhalb dem angegebenen Temperaturbereich wird gegebenenfalls bei den Siedetemperaturen des Reaktionsgemisches gearbeitet.

Als Lösungs- oder Verdünnungsmittel kommen unter den Reaktionsbedingungen inerte Lösungs- oder Verdünnungsmittel, wie aromatische Kohlenwasserstoffe, beispielsweise Toluol oder Xylol, aliphatische oder aromatische Chlorkohlenwasserstoffe, wie Methylenchlorid, Ethylenchlorid oder Chlorbenzol, offenkettige oder cyclische aliphatische Ether, wie Diethylether, Tetrahydrofuran oder Dioxan, Dialkylketone, wie Aceton oder Diethylketon, oder Dialkylformamide, wie Dimethylformamid, in Betracht. Gegebenenfalls kann ein flüssige Acylierungsmittel (IV) im Überschuß als Lösungs- oder Verdünnungsmittel verwendet werden. Auch Wasser oder ein Gemisch aus Wasser und einem unter den Reaktionsbedingungen inerten, mit Wasser mischbaren Lösungsmittel, wie Aceton, können verwendet werden. In diesem Fall können aber nur Acylierungsmittel eingesetzt werden, die schwer hydrolysieren.

Bei der Durchführung der Acylierung in einem Zwei-Phasensystem kommen als mit Wasser nicht mischbare Lösungsmittel unter den Reaktionsbedingungen inerte, mit Wasser nicht mischbare Lösungsmittel, wie aromatische Kohlenwasserstoffe, beispielsweise Toluol oder Xylol, aliphatische oder aromatische Chlorkohlenwasserstoffe, wie Methylenchlorid, Ethylenchlorid oder Chlorbenzol, oder aliphatische Ether, wie Diethylether, in Betracht. Wird im Zwei-Phasensystem mit einem leicht hydrolysierbaren Acylierungsmittel gearbeitet, so ist es zweckmäßig, die Verbindung der Formel (III) vorzulegen und, falls eine Base zur Anwendung kommt, auch diese vorzulegen oder gleichzeitig mit dem Acylierungsmittel zuzugeben.

Basen als säurebindende Mittel sind zweckmäßigerweise anorganische Basen, wie Natrium- oder Kaliumhydroxid, Natrium- oder Kaliumcarbonat, Natrium- oder Kaliumhydrogencarbonat, oder tertiäre organische Amine, insbesondere Triethylamin.

Die Acylierung einer Verbindung der Formel (III) mit einem Carbonsäureanhydrid kann in Gegenwart einer Mineralsäure, wie Schwefelsäure oder Perchlorsäure, als Katalysator erfolgen.

Die freien Verbindungen der Formel (III), in denen $R^2$ eine Alkoxygruppe bedeutet, können aus ihren Säureadditionssalzen bei Temperaturen von 0 bis 30°C, bevorzugt 0 bis 15°C, in üblicher Weise, z. B. durch Behandlung einer Lösung oder einer Suspension des Salzes in Wasser mit einer Base, wie Natriumhydroxid, freigesetzt und nach üblichen Methoden bei Temperaturen von 0 bis 30°C, bevorzugt 0 bis 15°C, isoliert werden. Zweckmäßiger ist es, auf diese Isolierung der freien Verbindungen (III) zu verzichten, ein Säureadditionssalz zu verwenden und dieses in an sich bekannter Weise im Reaktionsmedium mit einer Base, wie Natrium- oder Kaliumhydroxid, Natrium- oder Kaliumcarbonat, Natrium- oder Kaliumhydrogencarbonat, oder Triethylamin, in die freie Verbindung überzuführen und umzusetzen.

Auch für das zu acylierende 1,4-Dihydro-4-imino-1-phenylpyridazin (III, $R^2 = H$) gilt, daß es in Form der freien Base oder eines Säureadditionssalzes, das im Reaktionsmedium mit einer Base, wie Natrium- oder Kaliumhydroxid, Natrium- oder Kaliumcarbonat, Natrium- oder Kaliumhydrogencarbonat, oder Triethylamin, in die freie Verbindung übergeführt wird, eingesetzt werden kann.

Die direkte Acylierung eines Säureadditionssalzes einer Verbindung der Formel (III) mit einem Acylierungsmittel der Formel (IV) wird ebenfalls unter an sich üblichen Bedingungen durchgeführt. In der Regel unter Verwendung von wenigstens einer äquimolaren Menge des Acylierungsmittels, zweckmäßig in Gegenwart eines Lösungs- oder Verdünnungsmittels, bei Temperaturen zwischen 20 und 180°C, gegebenenfalls bei den Siedetemperatur des Reaktionsgemisches, und gegebenenfalls unter Anwendung von Druck.

Als Lösungs- oder Verdünnungsmittel kommen unter den Reaktionsbedingungen inerte Lösungs- oder Verdünnungsmittel, wie aromatische Kohlenwasserstoffe, beispielsweise Toluol oder Xylol, aliphatische oder aromatische Chlorwasserstoffe, wie Ethylenchlorid oder Chlorbenzol, offenkettige oder cyclische aliphatische Ether, wie Dibutylether oder Dioxan, Ketone, wie Diethylketon, oder Dialkylformamide, wie Dimethylformamid, in Betracht. Gegebenenfalls kann die dem Acylierungsmittel zugrundeliegende Säure als Lösungs- oder Verdünnungsmittel verwendet werden. Gegebenenfalls kann ein flüssiges Acylierungsmittel (IV) im Überschuß als Lösungs- oder Verdünnungsmittel verwendet werden.

Bei Verwendung eines Carbonsäureanhydrids als Acylierungsmittel kann in Gegenwart einer Mineralsäure, wie Schwefelsäure oder Perchlorsäure, als Katalysator gearbeitet werden.

Die erfindungsgemäßen Pyridazinonimine der Formel (I), in denen $R^1$ für eine Gruppe $-CO-Y-R^4$ steht, und ihre Säureadditionssalze mit einer physiologisch verträglichen Säure können hergestellt werden, indem man eine Verbindung der Formel (III), in der $R^2$ die für Formel (I) angegebenen Bedeutungen hat, gegebenenfalls in Form eines beliebigen Säureadditionssalzes, mit einer Verbindung der Formel (V),

$$R^4—Y—CO—B \qquad (V)$$

in der $R^4$ und Y wie oben definiert sind und B für ein Halogenatom, insbesondere ein Chloratom, steht, in an sich bekannter Weise umsetzt und die erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

Die Umsetzung einer Verbindung der Formel (III) oder eines ihrer Säureadditionssalze mit einem Halogenameisensäureester oder Halogenthioameisensäure-S-ester (V) wird unter den oben für die Acylierung einer Verbindung der Formel (III) oder eines ihrer Säureadditionssalze mit einem Carbonsäurehalogenid der Formel (IV) (A = Halogen) angegebenen Bedingungen durchgeführt.

Die erfindungsgemäßen Pyridazinonimine der Formel (I), in denen $R^1$ für eine Gruppe

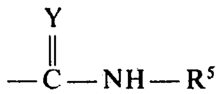

steht, und ihre Säureadditionssalze mit einer physiologisch verträglichen Säure können hergestellt werden, indem man eine Verbindung der Formel (III), in der $R^2$ die für die Formel (I) angegebenen Bedeutungen hat, gegebenenfalls in Form eines beliebigen Säureadditionssalzes, mit einer Verbindung der Formel (VI),

$$R^5—N{=}C{=}Y \qquad (VI)$$

in der $R^5$ und Y wie oben definiert sind, in an sich bekannter Weise umsetzt und die dabei erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

Die Umsetzung einer Verbindung der Formel (III) oder eines ihrer Säureadditionssalze mit einem Isocyanat oder Isothiocyanat (VI) wird unter den oben für die Acylierung einer Verbindung der Formel (III) oder eines ihrer Säureadditionssalze mit einem Carbonsäurehalogenid der Formel (IV) (A = Halogen) angegebenen Bedingungen durchgeführt. Natürlich kommt aber bei der Reaktion zwischen einer Verbindung der Formel (III) und einem Isocyanat bzw. Isothiocyanat keine Hilfsbase zur Anwendung.

Zur Herstellung der Verbindungen (I), in denen $R^1$ eine Gruppe

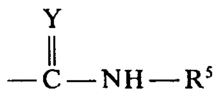

bedeutet, und ihrer Säureadditionssalze mit einer physiologisch verträglichen Säure läßt man das Isocyanat oder Isothiocyanat (VI) bevorzugt auf eine freie Verbindung einwirken und führt das dabei als freies Imin erhaltene Reaktionsprodukt gegebenenfalls in das Säureadditionssalz einer physiologisch verträglichen Säure über. Die Umsetzung mit einer Verbindung (VI) kann dabei unter Zusatz eines der zur Beschleunigung von Isocyanatreaktionen üblicherweise eingesetzten Katalysator, wie Triethylamin, erfolgen.

Die erfindungsgemäßen Pyridazinonimine der Formel (I), in denen $R^1$ für eine Gruppe $-CO-COO-R^6$ steht, und ihre Säureadditionssalze mit einer physiologisch verträglichen Säure können hergestellt werden, indem man eine Verbindung der Formel (III), in der $R^2$ die für die Formel (I) angegebenen Bedeutungen hat, gegebenenfalls in Form eines beliebigen Säureadditionssalzes, mit einem Oxalsäureesterchlorid der Formel (VII),

$$R^6—OOC—CO—Cl \qquad (VII)$$

in der $R^6$ wie oben definiert ist, in an sich bekannter Weise umsetzt und die erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

Zur Umsetzung einer Verbindung der Formel (III) oder eines ihrer Säureadditionssalze mit einem Oxalsäureesterchlorid (VII) wird unter den oben für die Acylierung einer Verbindung der Formel (III) oder eines ihrer Säureadditionssalze mit einem Carbonsäurehalogenid der Formel (IV) (A = Halogen) angegebenen Bedingungen gearbeitet.

Die Umwandlung einer als freies Imin erhaltenen erfindungsgemäßen Verbindung in das Säureadditionssalz einer physiologisch verträglichen Säure wird, wie oben angegeben, nach üblichen Methoden durchgeführt. In der Regel durch Umsetzung der freien Base mit der entsprechenden physiologisch verträglichen Säure. Diese Umsetzung kann z. B. durch Mischen der Base mit der Säure in Wasser, in einem organischen Lösungsmittel, beispielsweise einem niederen Alkohol, wie Methanol oder Ethanol, einem Keton, wie Aceton oder Diethylketon, einem Chlorkohlenwasserstoff, wie Methylenchlorid oder Ethylenchlorid, oder einem Ether, wie Diethylether, Tetrahydrofuran oder Dioxan, in Mischungen der genannten organischen Lösungsmittel, in Mischungen von Wasser und den genannten, mit Wasser mischbaren organischen Lösungsmitteln oder in einem Zwei-Phasensystem, bestehend aus Wasser

und einem der genannten, mit Wasser nicht mischbaren organischen Lösungsmittel, erfolgen. Es können auch z. B. Lösungen der freien Base in den genannten organischen Lösungsmitteln mit Lösungen der Säure in Wasser oder den genannten organischen Lösungsmitteln gemischt werden.

Eine als Säureadditionssalz erhaltene erfindungsgemäße Verbindung kann, wie oben angegeben, in an sich bekannter Weise, z. B. mit Basen oder Ionenaustauschern, in die freie Verbindung übergeführt werden.

Die Umwandlung eines Säureadditionssalzes einer Verbindung der Formel (I) in das Säureadditionssalz einer anderen, physiologisch verträglichen, Säure wird ebenfalls, wie oben angegeben, nach bekannten Verfahren durchgeführt. Zum Beispiel durch Überführung des ursprünglich erhaltenen Salzes in die freie Verbindung und deren Umwandlung in das gewünschte Säureadditionssalz oder durch Austausch des Anions des ursprünglich erhaltenen Salzes gegen das Anion einer anderen, physiologisch verträglichen, Säure mittels Ionenaustauscher.

Die als Ausgangsverbindungen verwendeten Säureadditionssalze der Pyridazinonimine der Formel (III) sind gemäß DE-A-1 912 941 oder 2 245 248 bekannt oder können nach den darin beschriebenen Methoden hergestellt werden. Das als Ausgangssubstanz verwendete 1,4-Dihydro-4-imino-1-phenylpyridazin kann aus seinen Säureadditionssalzen, die in der DE-A-2 245 248 beschrieben werden, durch Behandeln eines Salzes in Wasser mit Natriumhydroxid hergestellt werden. Die Verbindung ist kristallin und besitzt einen Schmelzpunkt von 97 bis 98°C (aus Cyclohexan).

Erfindungsgemäße Verbindungen, die nach den genannten Verfahren erhalten werden, sind beispielsweise:

4-Acetylimino-1,4-dihydro-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-(tert-Butoxy)-, 6-Pentyloxy-, 6-Hexyloxy-, 6-Heptyloxy- und 6-Octyloxy-4-acetylimino-1,4-dihydro-1-phenylpyridazin;
1,4-Dihydro-1-phenyl-4-(propionylimino)pyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy-, 6-Hexyloxy-, 6-Heptyloxy- und 6-Octyloxy-1,4-dihydro-1-phenyl-4-(propionylimino)pyridazin;
4-Butyrylimino-1,4-dihydro-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy- und 6-Octyloxy-4-butyrylimino-1,4-dihydro-1-phenylpyridazin;
1,4-Dihydro-4-isobutyrylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-isobutyrylimino-1-phenylpyridazin;
1,4-Dihydro-1-phenyl-4-(valerylimino)pyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-1-phenyl-4-(valerylimino)pyridazin;
1,4-Dihydro-4-(2-methylbutyryl)imino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-(2-methylbutyryl)imino-1-phenylpyridazin;
1,4-Dihydro-4-isovalerylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-isovalerylimino-1-phenylpyridazin;
1,4-Dihydro-1-phenyl-4-(pivaloylimino)pyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-1-phenyl-4-(pivaloylimino)pyridazin;
1,4-Dihydro-4-hexanoylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-hexanoylimino-1-phenylpyridazin;
1,4-Dihydro-4-heptanoylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-heptanoylimino-1-phenylpyridazin;
1,4-Dihydro-4-octanoylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-octanoylimino-1-phenylpyridazin;
1,4-Dihydro-4-nonanoylimino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-nonanoylimino-1-phenylpyridazin;
1,4-Dihydro-1-phenyl-4-[(phenylacetyl)imino]pyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy-, 6-Hexyloxy- und 6-Octyloxy-1,4-dihydro-1-phenyl-4-[(phenylacetyl)imino]pyridazin;
1,4-Dihydro-1-phenyl-4-[(o-tolylacetyl)imino]pyridazin;
6-Methoxy-, 6-Ethoxy-, 6-Propoxy-, 6-Isopropoxy-, 6-Butoxy- und 6-Pentyloxy-1,4-dihydro-1-

phenyl-4-[(o-tolylacetyl)imino]pyridazin;
1,4-Dihydro-1-phenyl-4-[(m-tolylacetyl)imino]pyridazin;
6-Methoxy-, 6-Ethoxy-, 6-Propoxy-, 6-Isopropoxy-, 6-Butoxy- und 6-Pentyloxy-1,4-dihydro-1-phenyl-4-[(m-tolylacetyl)imino]pyridazin;
1,4-Dihydro-1-phenyl-4-[(p-tolylacetyl)imino]pyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-1-phenyl-4-[(p-tolylacetyl)imino]pyridazin;
1,4-Dihydro-4-(o,p-dimethylphenylacetyl)imino-1-phenylpyridazin;
6-Methoxy-, 6-Ethoxy-, 6-Propoxy-, 6-Isopropoxy- und 6-Butoxy-1,4-dihydro-4-(o,p-dimethylphenylacetyl)imino-1-phenylpyridazin;
1,4-Dihydro-4-(o-methoxyphenylacetyl)imino-1-phenylpyridazin;
6-Methoxy-, 6-Ethoxy-, 6-Propoxy-, 6-Isopropoxy-, 6-Butoxy- und 6-Isobutoxy-1,4-dihydro-4-(o-methoxyphenylacetyl)imino-1-phenylpyridazin;
1,4-Dihydro-4-(m-methoxyphenylacetyl)imino-1-phenylpyridazin;
6-Methoxy-, 6-Ethoxy-, 6-Propoxy-, 6-Isopropoxy-, 6-Butoxy- und 6-Isobutoxy-1,4-dihydro-4-(m-methoxyphenylacetyl)imino-1-phenylpyridazin;
1,4-Dihydro-4-(p-methoxyphenylacetyl)imino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-(p-Methoxyphenylacetyl)imino-1-phenylpyridazin;
1,4-Dihydro-4-[(2,5-dimethoxyphenyl)acetyl]imino-1-phenylpyridazin;
6-Methoxy-, 6-Ethoxy-, 6-Propoxy-, 6-Isopropoxy-, 6-Butoxy- und 6-Isobutoxy-1,4-dihydro-4-[(2,5-dimethoxyphenyl)acetyl]imino-1-phenylpyridazin;
1,4-Dihydro-4-[(3,4-dimethoxyphenyl)acetyl]imino-1-phenylpyridazin;
6-Methoxy-, 6-Ethoxy-, 6-Propoxy-, 6-Isopropoxy-, 6-Butoxy-, 6-Isobutoxy- und 6-Pentyloxy-1,4-dihydro-4-[(3,4-dimethoxyphenyl)acetyl]imino-1-phenylpyridazin;
1,4-Dihydro-1-phenyl-4-[(m,m',p-trimethoxyphenylacetyl)-imino]pyridazin;
6-Methoxy-, 6-Ethoxy-, 6-Propoxy-, und 6-Butoxy-1,4-dihydro-1-phenyl-4-[(m,m',p-trimethoxyphenylacetyl)imino]pyridazin;
4-(o-Chlorphenylacetat)imino-1,4-dihydro-1-phenylpyridazin;
6-Methoxy-, 6-Ethoxy-, 6-Propoxy-, 6-Isopropoxy-, 6-Butoxy- und 6-Isobutoxy-4-(o-chlorphenylacetyl)imino-1,4-dihydro-1-phenylpyridazin;
4-(m-Chlorphenylacetyl)imino-1,4-dihydro-1-phenylpyridazin;
6-Methoxy-, 6-Ethoxy-, 6-Propoxy-, 6-Isopropoxy-, 6-Butoxy-, 6-Isobutoxy- und 6-Pentyloxy-4-(m-chlorphenylacetyl)imino-1,4-dihydro-1-phenylpyridazin;
4-(p-Chlorphenylacetyl)imino-1,4-dihydro-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-4-(p-chlorphenylacetyl)imino-1,4-dihydro-1-phenylpyridazin;
4-(o,p-Dichlorphenylacetyl)imino-1,4-dihydro-1-phenylpyridazin;
6-Methoxy-, 6-Ethoxy-, 6-Propoxy-, 6-Isopropoxy-, 6-Butoxy- und 6-Pentyloxy-4-(o,p-dichlorphenylacetyl)imino-1,4-dihydro-1-phenylpyridazin;
4-(m,p-Dichlorphenylacetyl)imino-1,4-dihydro-1-phenylpyridazin;
6-Methoxy-, 6-Ethoxy-, 6-Propoxy-, 6-Isopropoxy-, 6-Butoxy- und 6-Pentyloxy-4-(m,p-dichlorphenylacetyl)imino-1,4-dihydro-1-phenylpyridazin;
1,4-Dihydro-4-(o-fluorphenylacetyl)imino-1-phenylpyridazin;
6-Methoxy-, 6-Ethoxy-, 6-Propoxy-, 6-Isopropoxy-, 6-Butoxy-, 6-Isobutoxy- und 6-Pentyloxy-1,4-dihydro-4-(o-fluorphenylacetyl)imino-1-phenylpyridazin;
1,4-Dihydro-4-(p-fluorphenylacetyl)imino-1-phenylpyridazin;
6-Methoxy-, 6-Ethoxy-, 6-Propoxy-, 6-Isopropoxy-, 6-Butoxy-, 6-Isobutoxy-, 6-Pentyloxy- und 6-Octyloxy-1,4-dihydro-4-(p-fluorphenylacetyl)imino-1-phenylpyridazin;
4-(o-Chlor-o'-fluorphenylacetyl)imino-1,4-dihydro-1-phenylpyridazin;
6-Methoxy-, 6-Ethoxy-, 6-Propoxy- und 6-Butoxy-4-(o-chlor-o'-fluorphenylacetyl)imino-1,4-dihydro-1-phenylpyridazin;
1,4-Dihydro-1-phenyl-4-{[m-(trifluormethyl)phenylacetyl]-imino}pyridazin;
6-Methoxy-, 6-Ethoxy-, 6-Propoxy-, 6-Isopropoxy-, 6-Butoxy-, 6-Isobutoxy- und 6-Pentyloxy-1,4-dihydro-1-phenyl-4-{[m-(trifluormethyl)phenylacetyl]imino}pyridazin;
4-(Cyclopropylcarbonyl)imino-1,4-dihydro-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy- und 6-Octyloxy-4-(cyclopropylcarbonyl)imino-1,4-dihydro-1-phenylpyridazin;
1,4-Dihydro-4-[(1-methylcyclopropyl)carbonyl]imino-1-phenylpyridazin;
6-Methoxy-, 6-Ethoxy-, 6-Propoxy-, 6-Isopropoxy-, 6-Butoxy-, 6-Isobutoxy- und 6-Pentyloxy-1,4-dihydro-4-[(1-methylcyclopropyl)carbonyl]imino-1-phenylpyridazin;
1,4-Dihydro-4-[(2-methylcyclopropyl)carbonyl]imino-1-phenylpyridazin;
6-Methoxy-, 6-Ethoxy-, 6-Propoxy-, 6-Isopropoxy-, 6-Butoxy-, 6-Isobutoxy- und 6-Pentyloxy-1,4-dihydro-4-[(2-methylcyclopropyl)carbonyl]imino-1-phenylpyridazin;
1,4-Dihydro-4-[(2,2-dimethylcyclopropyl)carbonyl]imino-1-phenylpyridazin;

6-Methoxy-, 6-Ethoxy-, 6-Propoxy-, 6-Isopropoxy-, 6-Butoxy-, 6-Isobutoxy- und 6-Pentyloxy-1,4-dihydro-4-[(2,2-dimethylcyclopropyl)carbonyl]imino-1-phenylpyridazin;
4-(Cyclobutylcarbonyl)imino-1,4-dihydro-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy-, 6-Hexyloxy- und 6-Octyloxy-4-(cyclobutylcarbonyl)imino-1,4-dihydro-1-phenylpyridazin;
1,4-Dihydro-4-[(1-methylcyclobutyl)carbonyl]imino-1-phenylpyridazin;
6-Methoxy-, 6-Ethoxy-, 6-Propoxy-, 6-Butoxy- und 6-Pentyloxy-1,4-dihydro-4-[(1-methylcyclobutyl)carbonyl]imino-1-phenylpyridazin;
4-(Cyclopentylcarbonyl)imino-1,4-dihydro-1-phenylpyridazin;
6-Methoxy-, 6-Ethoxy-, 6-Propoxy-, 6-Isopropoxy-, 6-Butoxy-, 6-Isobutoxy- und 6-Pentyloxy-4-(cyclopentylcarbonyl)imino-1,4-dihydro-1-phenylpyridazin;
4-(Cyclohexylcarbonyl)imino-1,4-dihydro-1-phenylpyridazin;
6-Methoxy-, 6-Ethoxy-, 6-Propoxy-, 6-Isopropoxy-, 6-Butoxy- und 6-Isobutoxy-4-(cyclohexylcarbonyl)imino-1,4-dihydro-1-phenylpyridazin;
1,4-Dihydro-4-[(1-methylcyclohexyl)carbonyl]imino-1-phenylpyridazin;
6-Methoxy-, 6-Ethoxy-, 6-Propoxy- und 6-Butoxy-1,4-dihydro-4-[(1-methylcyclohexyl)carbonyl]imino-1-phenylpyridazin;
4-(Cycloheptylcarbonyl)imino-1,4-dihydro-1-phenylpyridazin;
6-Methoxy-, 6-Ethoxy-, 6-Propoxy-, 6-Isopropoxy- und 6-Butoxy-4-(cycloheptylcarbonyl)imino-1,4-dihydro-1-phenylpyridazin;
4-(Cyclooctylcarbonyl)imino-1,4-dihydro-1-phenylpyridazin;
6-Methoxy-, 6-Ethoxy- und 6-Propoxy-4-(cyclooctylcarbonyl)imino-1,4-dihydro-1-phenylpyridazin;
4-Acryloylimino-1,4-dihydro-1-phenylpyridazin;
6-Methoxy-, 6-Ethoxy-, 6-Propoxy-, 6-Isopropoxy-, 6-Butoxy- und 6-Octyloxy-4-acryloylimino-1,4-dihydro-1-phenylpyridazin;
1,4-Dihydro-4-methacryloylimino-1-phenylpyridazin;
6-Methoxy-, 6-Ethoxy-, 6-Propoxy- und 6-Butoxy-1,4-dihydro-4-methacryloylimino-1-phenylpyridazin;
4-Crotonoylimino-1,4-dihydro-1-phenylpyridazin;
6-Methoxy-, 6-Ethoxy-, 6-Propoxy- und 6-Butoxy-4-crotonoylimino-1,4-dihydro-1-phenylpyridazin;
1,4-Dihydro-1-phenyl-4-[(vinylacetyl)imino]pyridazin;
6-Methoxy-, 6-Ethoxy- und 6-Propoxy-1,4-dihydro-1-phenyl-4-[(vinylacetyl)imino]pyridazin;
trans-1,4-Dihydro-4-(2-methylbut-2-enoyl)imino-1-phenylpyridazin;
6-Methoxy-, 6-Ethoxy- und 6-Propoxy-trans-1,4-dihydro-4-(2-methylbut-2-enoyl)imino-1-phenylpyridazin;
1,4-Dihydro-4-(3,3-dimethylacryloyl)imino-1-phenylpyridazin;
6-Methoxy-, 6-Ethoxy- und 6-Propoxy-1,4-dihydro-4-(3,3-dimethylacryloyl)imino-1-phenylpyridazin;
trans-1,4-Dihydro-4-(hex-2-enoyl)imino-1-phenylpyridazin;
6-Methoxy-, 6-Ethoxy- und 6-Propoxy-trans-1,4-dihydro-4-(hex-2-enoyl)imino-1-phenylpyridazin;
trans-1,4-Dihydro-4-(hex-3-enoyl)imino-1-phenylpyridazin;
6-Methoxy- und 6-Ethoxy-trans-1,4-dihydro-4-(hex-3-enoyl)imino-1-phenylpyridazin;
4-Benzoylimino-1,4-dihydro-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy- und 6-Octyloxy-4-benzoylimino-1,4-dihydro-1-phenylpyridazin;
1,4-Dihydro-1-phenyl-4-[(2-toluoyl)imino]pyridazin;
6-Methoxy-, 6-Ethoxy- und 6-Propoxy-1,4-dihydro-1-phenyl-4-[(2-toluoyl)imino]pyridazin;
1,4-Dihydro-1-phenyl-4-[(3-toluoyl)imino]pyridazin;
6-Methoxy-, 6-Ethoxy- und 6-Propoxy-1,4-dihydro-1-phenyl-4-[(3-toluoyl)imino]pyridazin;
1,4-Dihydro-1-phenyl-4-[(4-toluoyl)imino]pyridazin;
6-Methoxy-, 6-Ethoxy- und 6-Propoxy-1,4-dihydro-1-phenyl-4-[(4-toluoyl)imino]pyridazin;
1,4-Dihydro-4-(2,4-dimethylbenzoyl)imino-1-phenylpyridazin;
6-Methoxy- und 6-Ethoxy-1,4-dihydro-4-(2,4-dimethylbenzoyl)imino-1-phenylpyridazin;
1,4-Dihydro-4-(2-methoxybenzoyl)imino-1-phenylpyridazin;
6-Methoxy-, 6-Ethoxy- und 6-Propoxy-1,4-dihydro-4-(2-methoxybenzoyl)imino-1-phenylpyridazin;
1,4-Dihydro-4-(3-methoxybenzoyl)imino-1-phenylpyridazin;
6-Methoxy-, 6-Ethoxy-, 6-Propoxy- und 6-Butoxy-1,4-dihydro-4-(3-methoxybenzoyl)imino-1-phenylpyridazin;
1,4-Dihydro-4-(4-methoxybenzoyl)imino-1-phenylpyridazin;
6-Methoxy-, 6-Ethoxy-, 6-Propoxy- und 6-Butoxy-1,4-dihydro-4-(4-methoxybenzoyl)imino-1-phenylpyridazin;
1,4-Dihydro-4-(2,3-dimethoxybenzoyl)imino-1-phenylpyridazin;

6-Methoxy-, 6-Ethoxy-, 6-Propoxy- und 6-Butoxy-1,4-dihydro-4-(2,3-dimethoxybenzoyl)imino-1-phenylpyridazin;
1,4-Dihydro-4-(2,4-dimethoxybenzoyl)imino-1-phenylpyridazin;
6-Methoxy-, 6-Ethoxy-, 6-Propoxy- und 6-Butoxy-1,4-dihydro-4-(2,4-dimethoxybenzoyl)imino-1-phenylpyridazin;
1,4-Dihydro-4-(2,6-dimethoxybenzoyl)imino-1-phenylpyridazin;
6-Methoxy-, 6-Ethoxy- und 6-Propoxy-1,4-dihydro-4-(2,6-dimethoxybenzoyl)imino-1-phenylpyridazin;
1,4-Dihydro-4-(3,4-dimethoxybenzoyl)imino-1-phenylpyridazin;
6-Methoxy-, 6-Ethoxy-, 6-Propoxy- und 6-Butoxy-1,4-dihydro-4-(3,4-dimethoxybenzoyl)imino-1-phenylpyridazin;
1,4-Dihydro-4-(3,5-dimethoxybenzoyl)imino-1-phenylpyridazin;
6-Methoxy-, 6-Ethoxy-, 6-Propoxy-, 6-Butoxy- und 6-Octyloxy-1,4-dihydro-4-(3,5-dimethoxybenzoyl)imino-1-phenylpyridazin;
1,4-Dihydro-1-phenyl-4-[(3,4,5-trimethoxybenzoyl)imino]-pyridazin;
6-Methoxy- und 6-Ethoxy-1,4-dihydro-1-phenyl-4-[(3,4,5-trimethoxybenzoyl)imino]pyridazin;
4-(2-Chlorbenzoyl)imino-1,4-dihydro-1-phenylpyridazin;
6-Methoxy-, 6-Ethoxy- und 6-Propoxy-4-(2-chlorbenzoyl)imino-1,4-dihydro-1-phenylpyridazin;
4-(3-Chlorbenzoyl)imino-1,4-dihydro-1-phenylpyridazin;
6-Methoxy-, 6-Ethoxy-, 6-Propoxy- und 6-Butoxy-4-(3-chlorbenzoyl)imino-1,4-dihydro-1-phenylpyridazin;
4-(4-Chlorbenzoyl)imino-1,4-dihydro-1-phenylpyridazin;
6-Methoxy-, 6-Ethoxy-, 6-Propoxy-, 6-Isopropoxy- und 6-Butoxy-4-(4-chlorbenzoyl)imino-1,4-dihydro-1-phenylpyridazin;
1,4-Dihydro-4-(2-fluorbenzoyl)imino-1-phenylpyridazin;
6-Methoxy-, 6-Ethoxy- und 6-Propoxy-1,4-dihydro-4-(2-fluorbenzoyl)imino-1-phenylpyridazin;
1,4-Dihydro-4-(3-fluorbenzoyl)imino-1-phenylpyridazin;
6-Methoxy-, 6-Ethoxy- und 6-Propoxy-1,4-dihydro-4-(3-fluorbenzoyl)imino-1-phenylpyridazin;
1,4-Dihydro-4-(4-fluorbenzoyl)imino-1-phenylpyridazin;
6-Methoxy-, 6-Ethoxy-, 6-Propoxy- und 6-Butoxy-1,4-dihydro-4-(4-fluorbenzoyl)imino-1-phenylpyridazin;
4-(2,3-Dichlorbenzoyl)imino-1,4-dihydro-1-phenylpyridazin;
6-Methoxy-, 6-Ethoxy-, 6-Propoxy- und 6-Butoxy-4-(2,3-dichlorbenzoyl)imino-1,4-dihydro-1-phenylpyridazin;
4-(2,4-Dichlorbenzoyl)imino-1,4-dihydro-1-phenylpyridazin;
6-Methoxy-, 6-Ethoxy-, 6-Propoxy-, 6-Isopropoxy-, 6-Butoxy-, 6-Isobutoxy- und 6-Octyloxy-4-(2,4-dichlorbenzoyl)imino-1,4-dihydro-1-phenylpyridazin;
4-(2,5-Dichlorbenzoyl)imino-1,4-dihydro-1-phenylpyridazin;
6-Methoxy-, 6-Ethoxy-, 6-Propoxy- und 6-Butoxy-4-(2,5-dichlorbenzoyl)imino-1,4-dihydro-1-phenylpyridazin;
4-(2,6-Dichlorbenzoyl)imino-1,4-dihydro-1-phenylpyridazin;
6-Methoxy-, 6-Ethoxy-, 6-Propoxy- und 6-Butoxy-4-(2,6-dichlorbenzoyl)imino-1,4-dihydro-1-phenylpyridazin;
4-(3,4-Dichlorbenzoyl)imino-1,4-dihydro-1-phenylpyridazin;
6-Methoxy-, 6-Ethoxy-, 6-Propoxy- und 6-Butoxy-4-(3,4-dichlorbenzoyl)imino-1,4-dihydro-1-phenylpyridazin;
4-(3,5-Dichlorbenzoyl)imino-1,4-dihydro-1-phenylpyridazin;
6-Methoxy-, 6-Ethoxy-, 6-Propoxy- und 6-Butoxy-4-(3,5-dichlorbenzoyl)imino-1,4-dihydro-1-phenylpyridazin;
4-(2,5-Difluorbenzoyl)imino-1,4-dihydro-1-phenylpyridazin;
6-Methoxy-, 6-Ethoxy-, 6-Propoxy- und 6-Butoxy-4-(2,5-difluorbenzoyl)imino-1,4-dihydro-1-phenylpyridazin;
1,4-Dihydro-1-phenyl-4-{[2-(trifluormethyl)benzoyl]imino}pyridazin;
6-Methoxy-, 6-Ethoxy- und 6-Propoxy-1,4-dihydro-1-phenyl-4-{[2-(trifluormethyl)benzoyl]-imino}pyridazin;
1,4-Dihydro-1-phenyl-4-{[3-(trifluormethyl)benzoyl]imino}pyridazin;
6-Methoxy-, 6-Ethoxy-, 6-Propoxy- und 6-Butoxy-1,4-dihydro-1-phenyl-4-{[3-(trifluormethyl)-benzoyl]imino}pyridazin;
1,4-Dihydro-1-phenyl-4-{[4-(trifluormethyl)benzoyl]imino}pyridazin;
6-Methoxy-, 6-Ethoxy-, 6-Propoxy- und 6-Butoxy-1,4-dihydro-1-phenyl-4-{[4-(trifluormethyl)-benzoyl]imino}pyridazin;
1,4-Dihydro-4-(methoxycarbonyl)imino-1-phenylpyridazin;
6-Methoxy-, 6-Ethoxy-, 6-Propoxy-, 6-Butoxy- und 6-Octyloxy-1,4-dihydro-4-(methoxycarbonyl)imino-1-phenylpyridazin;
1,4-Dihydro-4-(ethoxycarbonyl)imino-1-phenylpyridazin;

6-Methoxy-, 6-Ethoxy-, 6-Propoxy-, 6-Isopropoxy- und 6-Butoxy-1,4-dihydro-4-(ethoxycarbonyl)imino-1-phenylpyridazin;
1,4-Dihydro-1-phenyl-4-[(propoxycarbonyl)imino]pyridazin;
6-Methoxy-, 6-Ethoxy-, 6-Propoxy- und 6-Butoxy-1,4-dihydro-1-phenyl-4-[(propoxycarbonyl)-imino]pyridazin;
1,4-Dihydro-4-(isopropoxycarbonyl)imino-1-phenylpyridazin;
6-Methoxy-, 6-Ethoxy-, 6-Propoxy- und 6-Butoxy-1,4-dihydro-4-(isopropoxycarbonyl)imino-1-phenylpyridazin;
4-(Butoxycarbonyl)imino-1,4-dihydro-1-phenylpyridazin;
6-Methoxy-, 6-Ethoxy-, 6-Propoxy- und 6-Butoxy-4-(butoxycarbonyl)imino-1,4-dihydro-1-phenylpyridazin;
4-[(sec-Butoxy)carbonyl]imino-1,4-dihydro-1-phenylpyridazin;
6-Methoxy-, 6-Ethoxy-, 6-Propoxy- und 6-Butoxy-4-[(sec-Butoxy)carbonyl]imino-1,4-dihydro-1-phenylpyridazin;
1,4-Dihydro-4-(isobutoxycarbonyl)imino-1-phenylpyridazin;
6-Methoxy-, 6-Ethoxy-, 6-Propoxy- und 6-Butoxy-1,4-dihydro-4-(isobutoxycarbonyl)imino-1-phenylpyridazin;
1,4-Dihydro-4-(pentyloxycarbonyl)imino-1-phenylpyridazin;
6-Methoxy-, 6-Ethoxy-, 6-Propoxy- und 6-Butoxy-1,4-dihydro-4-(pentyloxycarbonyl)imino-1-phenylpyridazin;
1,4-Dihydro-4-(hexyloxycarbonyl)imino-1-phenylpyridazin;
6-Methoxy-, 6-Ethoxy-, 6-Propoxy- und 6-Butoxy-1,4-dihydro-4-(hexyloxycarbonyl)imino-1-phenylpyridazin;
1,4-Dihydro-4-(heptyloxycarbonyl)imino-1-phenylpyridazin;
6-Methoxy-, 6-Ethoxy-, 6-Propoxy- und 6-Butoxy-1,4-dihydro-4-(heptyloxycarbonyl)imino-1-phenylpyridazin;
1,4-Dihydro-4-(octyloxycarbonyl)imino-1-phenylpyridazin;
6-Methoxy-, 6-Ethoxy-, 6-Propoxy- und 6-Butoxy-1,4-dihydro-4-(octyloxycarbonyl)imino-1-phenylpyridazin;
4-(Benzyloxycarbonyl)imino-1,4-dihydro-1-phenylpyridazin;
6-Methoxy-, 6-Ethoxy-, 6-Propoxy-, 6-Butoxy- und 6-Hexyloxy-4-(benzyloxycarbonyl)imino-1,4-dihydro-1-phenylpyridazin;
1,4-Dihydro-4-(p-methylbenzyloxycarbonyl)imino-1-phenylpyridazin;
6-Methoxy-, 6-Ethoxy- und 6-Propoxy-1,4-dihydro-4-(p-methylbenzyloxycarbonyl)imino-1-phenylpyridazin;
1,4-Dihydro-4-(p-methoxybenzyloxycarbonyl)imino-1-phenylpyridazin;
6-Methoxy-, 6-Ethoxy- und 6-Propoxy-1,4-dihydro-4-(p-methoxybenzyloxycarbonyl)imino-1-phenylpyridazin;
1,4-Dihydro-4-(m,p-dimethoxybenzyloxycarbonyl)imino-1-phenylpyridazin;
6-Methoxy-, 6-Ethoxy- und 6-Propoxy-1,4-dihydro-4-(m,p-dimethoxybenzyloxycarbonyl)imino-1-phenylpyridazin;
4-(p-Chlorbenzyloxycarbonyl)imino-1,4-dihydro-1-phenylpyridazin;
6-Methoxy-, 6-Ethoxy-, 6-Propoxy- und 6-Butoxy-4-(p-chlorbenzyloxycarbonyl)imino-1,4-dihydro-1-phenylpyridazin;
4-(o,p-Dichlorbenzyloxycarbonyl)imino-1,4-dihydro-1-phenylpyridazin;
6-Methoxy-, 6-Ethoxy-, 6-Propoxy- und 6-Butoxy-4-(o,p-dichlorbenzyloxycarbonyl)imino-1,4-dihydro-1-phenylpyridazin;
1,4-Dihydro-4-(p-fluorbenzyloxycarbonyl)imino-1-phenylpyridazin;
6-Methoxy-, 6-Ethoxy- und 6-Propoxy-1,4-dihydro-4-(p-fluorbenzyloxycarbonyl)imino-1-phenylpyridazin;
1,4-Dihydro-1-phenyl-4-{[m-(trifluormethyl)benzyloxycarbonyl]imino}pyridazin;
6-Methoxy-, 6-Ethoxy- und 6-Propoxy-1,4-dihydro-1-phenyl-4-{[m-(trifluormethyl)benzyloxycarbonyl]imino}pyridazin;
4-(Allyloxycarbonyl)imino-1,4-dihydro-1-phenylpyridazin;
6-Methoxy-, 6-Ethoxy-, 6-Propoxy-, 6-Butoxy- und 6-Hexyloxy-4-(allyloxycarbonyl)imino-1,4-dihydro-1-phenylpyridazin;
4-(But-2-enyloxycarbonyl)imino-1,4-dihydro-1-phenylpyridazin;
6-Methoxy-, 6-Ethoxy- und 6-Propoxy-4-(but-2-enyloxycarbonyl)imino-1,4-dihydro-1-phenylpyridazin;
4-(But-3-enyloxycarbonyl)imino-1,4-dihydro-1-phenylpyridazin;
6-Methoxy-, 6-Ethoxy- und 6-Propoxy-4-(but-3-enyloxycarbonyl)imino-1,4-dihydro-1-phenylpyridazin;
1,4-Dihydro-4-(1-methylprop-2-enyloxycarbonyl)imino-1-phenylpyridazin;
6-Methoxy-, 6-Ethoxy- und 6-Propoxy-1,4-dihydro-4-(1-methylprop-2-enyloxycarbonyl)imino-1-phenylpyridazin;

1,4-Dihydro-4-(pent-4-enyloxycarbonyl)imino-1-phenylpyridazin;
6-Methoxy- und 6-Ethoxy-1,4-dihydro-4-(pent-4-enyloxycarbonyl)imino-1-phenylpyridazin;
1,4-Dihydro-4-(phenoxycarbonyl)imino-1-phenylpyridazin;
6-Methoxy-, 6-Ethoxy-, 6-Propoxy- und 6-Butoxy-1,4-dihydro-4-(phenoxycarbonyl)imino-1-phenylpyridazin;
1,4-Dihydro-1-phenyl-4-[(2-tolyloxycarbonyl)imino]pyridazin;
6-Methoxy-, 6-Ethoxy- und 6-Propoxy-1,4-dihydro-1-phenyl-4-[(2-tolyloxycarbonyl)imino]pyridazin;
1,4-Dihydro-1-phenyl-4-[(3-tolyloxycarbonyl)imino]pyridazin;
6-Methoxy-, 6-Ethoxy- und 6-Propoxy-1,4-dihydro-1-phenyl-4-[(3-tolyloxycarbonyl)imino]pyridazin;
1,4-Dihydro-1-phenyl-4-[(4-tolyloxycarbonyl)imino]pyridazin;
6-Methoxy-, 6-Ethoxy- und 6-Propoxy-1,4-dihydro-1-phenyl-4-[(4-tolyloxycarbonyl)imino]pyridazin;
1,4-Dihydro-4-(2-methoxyphenoxycarbonyl)imino-1-phenylpyridazin;
6-Methoxy-, 6-Ethoxy- und 6-Propoxy-1,4-dihydro-4-(2-methoxyphenoxycarbonyl)imino-1-phenylpyridazin;
1,4-Dihydro-4-(3-methoxyphenoxycarbonyl)imino-1-phenylpyridazin;
6-Methoxy-, 6-Ethoxy-, 6-Propoxy- und 6-Butoxy-1,4-dihydro-4-(3-methoxyphenoxycarbonyl)-imino-1-phenylpyridazin;
1,4-Dihydro-4-(4-methoxyphenoxycarbonyl)imino-1-phenylpyridazin;
6-Methoxy-, 6-Ethoxy-, 6-Propoxy- und 6-Butoxy-1,4-dihydro-4-(4-methoxyphenoxycarbonyl)-imino-1-phenylpyridazin;
1,4-Dihydro-4-(3,4-dimethoxyphenoxycarbonyl)imino-1-phenylpyridazin;
6-Methoxy-, 6-Ethoxy-, 6-Propoxy- und 6-Butoxy-1,4-dihydro-4-(3,4-dimethoxyphenoxycarbonyl)imino-1-phenylpyridazin;
1,4-Dihydro-4-(3,5-dimethoxyphenoxycarbonyl)imino-1-phenylpyridazin;
6-Methoxy-, 6-Ethoxy-, 6-Propoxy- und 6-Butoxy-1,4-dihydro-4-(3,5-dimethoxyphenoxycarbonyl)imino-1-phenylpyridazin;
4-(2-Chlorphenoxycarbonyl)imino-1,4-dihydro-1-phenylpyridazin;
6-Methoxy-, 6-Ethoxy- und 6-Propoxy-4-(2-chlorphenoxycarbonyl)imino-1,4-dihydro-1-phenylpyridazin;
4-(3-Chlorphenoxycarbonyl)imino-1,4-dihydro-1-phenylpyridazin;
6-Methoxy-, 6-Ethoxy-, 6-Propoxy- und 6-Butoxy-4-(3-chlorphenoxycarbonyl)imino-1,4-dihydro-1-phenylpyridazin;
4-(4-Chlorphenoxycarbonyl)imino-1,4-dihydro-1-phenylpyridazin;
6-Methoxy-, 6-Ethoxy-, 6-Propoxy- und 6-Butoxy-4-(4-chlorphenoxycarbonyl)imino-1,4-dihydro-1-phenylpyridazin;
4-(2,4-Dichlorphenoxycarbonyl)imino-1,4-dihydro-1-phenylpyridazin;
6-Methoxy-, 6-Ethoxy-, 6-Propoxy- und 6-Butoxy-4-(2,4-dichlorphenoxycarbonyl)imino-1,4-dihydro-1-phenylpyridazin;
4-(3,4-Dichlorphenoxycarbonyl)imino-1,4-dihydro-1-phenylpyridazin;
6-Methoxy-, 6-Ethoxy-, 6-Propoxy- und 6-Butoxy-4-(3,4-dichlorphenoxycarbonyl)imino-1,4-dihydro-1-phenylpyridazin;
1,4-Dihydro-4-(4-fluorphenoxycarbonyl)imino-1-phenylpyridazin;
6-Methoxy-, 6-Ethoxy- und 6-Propoxy-1,4-dihydro-4-(4-fluorphenoxycarbonyl)imino-1-phenylpyridazin;
1,4-Dihydro-1-phenyl-4-[3-(trifluormethyl)phenoxycarbonyl]imino pyridazin;
6-Methoxy-, 6-Ethoxy- und 6-Propoxy-1,4-dihydro-1-phenyl-4-{[3-(trifluormethyl)phenoxycarbonyl]imino}pyridazin;
1,4-Dihydro-4-(methylmercaptocarbonyl)imino-1-phenylpyridazin;
6-Methoxy-, 6-Ethoxy-, 6-Propoxy- und 6-Butoxy-1,4-dihydro-4-(methylmercaptocarbonyl)imino-1-phenylpyridazin;
1,4-Dihydro-4-(ethylmercaptocarbonyl)imino-1-phenylpyridazin;
6-Methoxy-, 6-Ethoxy-, 6-Propoxy- und 6-Butoxy-1,4-dihydro-4-(ethylmercaptocarbonyl)imino-1-phenylpyridazin;
1,4-Dihydro-1-phenyl-4-[(propylmercaptocarbonyl)imino]pyridazin;
6-Methoxy-, 6-Ethoxy- und 6-Propoxy-1,4-dihydro-1-phenyl-4-[(propylmercaptocarbonyl)imino]pyridazin;
1,4-Dihydro-4-(isopropylmercaptocarbonyl)imino-1-phenylpyridazin;
6-Methoxy-, 6-Ethoxy- und 6-Propoxy-1,4-dihydro-4-(isopropylmercaptocarbonyl)imino-1-phenylpyridazin;
4-(Butylmercaptocarbonyl)imino-1,4-dihydro-1-phenylpyridazin;
6-Methoxy-, 6-Ethoxy- und 6-Propoxy-4-(butylmercaptocarbonyl)imino-1,4-dihydro-1-phenylpyridazin;

1,4-Dihydro-4-(pentylmercaptocarbonyl)imino-1-phenylpyridazin;
6-Methoxy-, 6-Ethoxy- und 6-Propoxy-1,4-dihydro-4-(pentylmercaptocarbonyl)imino-1-phenylpyridazin;
1,4-Dihydro-4-(hexylmercaptocarbonyl)imino-1-phenylpyridazin;
6-Methoxy-, 6-Ethoxy- und 6-Propoxy-1,4-dihydro-4-(hexylmercaptocarbonyl)imino-1-phenylpyridazin;
1,4-Dihydro-4-(heptylmercaptocarbonyl)imino-1-phenylpyridazin;
6-Methoxy-, 6-Ethoxy- und 6-Propoxy-1,4-dihydro-4-(heptylmercaptocarbonyl)imino-1-phenylpyridazin;
4-(Benzylmercaptocarbonyl)imino-1,4-dihydro-1-phenylpyridazin;
6-Methoxy-, 6-Ethoxy-, 6-Propoxy- und 6-Butoxy-4-(benzylmercaptocarbonyl)imino-1,4-dihydro-1-phenylpyridazin;
1,4-Dihydro-4-(p-methylbenzylmercaptocarbonyl)imino-1-phenylpyridazin;
6-Methoxy- und 6-Ethoxy-1,4-dihydro-4-(p-methylbenzylmercaptocarbonyl)imino-1-phenylpyridazin;
1,4-Dihydro-4-(p-methoxybenzylmercaptocarbonyl)imino-1-phenylpyridazin;
6-Methoxy- und 6-Ethoxy-1,4-dihydro-4-(p-methoxybenzylmercaptocarbonyl)imino-1-phenylpyridazin;
4-(p-Chlorbenzylmercaptocarbonyl)imino-1,4-dihydro-1-phenylpyridazin;
6-Methoxy-, 6-Ethoxy- und 6-Propoxy-4-(p-chlorbenzylmercaptocarbonyl)imino-1,4-dihydro-1-phenylpyridazin;
1,4-Dihydro-4-(p-fluorbenzylmercaptocarbonyl)imino-1-phenylpyridazin;
6-Methoxy-, 6-Ethoxy- und 6-Propoxy-1,4-dihydro-4-(p-fluorbenzylmercaptocarbonyl)imino-1-phenylpyridazin;
1,4-Dihydro-1-phenyl-4-{[m-(trifluormethyl)benzylmercaptocarbonyl]imino}pyridazin;
6-Methoxy- und 6-Ethoxy-1,4-dihydro-1-phenyl-4-{[m-(trifluormethyl)benzylmercaptocarbonyl]imino}pyridazin;
4-(Allylmercaptocarbonyl)imino-1,4-dihydro-1-phenylpyridazin;
6-Methoxy-, 6-Ethoxy- und 6-Propoxy-4-(allylmercaptocarbonyl)imino-1,4-dihydro-1-phenylpyridazin;
4-(But-2-enylmercaptocarbonyl)imino-1,4-dihydro-1-phenylpyridazin;
6-Methoxy- und 6-Ethoxy-4-(but-2-enylmercaptocarbonyl)imino-1,4-dihydro-1-phenylpyridazin;
4-(But-3-enylmercaptocarbonyl)imino-1,4-dihydro-1-phenylpyridazin;
6-Methoxy- und 6-Ethoxy-4-(but-3-enylmercaptocarbonyl)imino-1,4-dihydro-1-phenylpyridazin;
1,4-Dihydro-1-phenyl-4-[(phenylmercaptocarbonyl)imino]-pyridazin;
6-Methoxy-, 6-Ethoxy- und 6-Propoxy-1,4-dihydro-1-phenyl-4-[(phenylmercaptocarbonyl)imino]pyridazin;
1,4-Dihydro-1-phenyl-4-[(4-tolylmercaptocarbonyl)imino]-pyridazin;
6-Methoxy- und 6-Ethoxy-1,4-dihydro-1-phenyl-4-[(4-tolylmercaptocarbonyl)imino]pyridazin;
1,4-Dihydro-4-(4-methoxyphenylmercaptocarbonyl)imino-1-phenylpyridazin;
6-Methoxy- und 6-Ethoxy-1,4-dihydro-4-(4-methoxyphenylmercaptocarbonyl)imino-1-phenylpyridazin;
4-(4-Chlorphenylmercaptocarbonyl)imino-1,4-dihydro-1-phenylpyridazin;
6-Methoxy-, 6-Ethoxy- und 6-Propoxy-4-(4-chlorphenylmercaptocarbonyl)imino-1,4-dihydro-1-phenylpyridazin;
1,4-Dihydro-4-(4-fluorphenylmercaptocarbonyl)imino-1-phenylpyridazin;
6-Methoxy- und 6-Ethoxy-1,4-dihydro-4-(4-fluorphenylmercaptocarbonyl)imino-1-phenylpyridazin;
1,4-Dihydro-1-phenyl-4-{[3-(trifluormethyl)phenylmercaptocarbonyl]imino}pyridazin;
6-Methoxy- und 6-Ethoxy-1,4-dihydro-1-phenyl-4-{[3-(trifluormethyl)phenylmercaptocarbonyl]imino}pyridazin;
1,4-Dihydro-4-(methylcarbamoyl)imino-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy- und 6-Octyloxy-1,4-dihydro-4-(methylcarbamoyl)imino-1-phenylpyridazin;
1,4-Dihydro-4-(ethylcarbamoyl)imino-1-phenylpyridazin;
6-Methoxy-, 6-Ethoxy-, 6-Propoxy-, 6-Isopropoxy-, 6-Butoxy- und 6-Pentyloxy-1,4-dihydro-4-(ethylcarbamoyl)imino-1-phenylpyridazin;
1,4-Dihydro-1-phenyl-4-[(propylcarbamoyl)imino]pyridazin;
6-Methoxy-, 6-Ethoxy-, 6-Propoxy-, 6-Isopropoxy- und 6-Butoxy-1,4-dihydro-1-phenyl-4-[(propylcarbamoyl)imino]pyridazin;
1,4-Dihydro-4-(isopropylcarbamoyl)imino-1-phenylpyridazin;
6-Methoxy-, 6-Ethoxy-, 6-Propoxy-, 6-Isopropoxy- und 6-Butoxy-1,4-dihydro-4-(isopropylcarbamoyl)imino-1-phenylpyridazin;

4-(Butylcarbamoyl)imino-1,4-dihydro-1-phenylpyridazin;
6-Methoxy-, 6-Ethoxy-, 6-Propoxy- und 6-Butoxy-4-(butylcarbamoyl)imino-1,4-dihydro-1-phenylpyridazin;
4-[(sec-Butyl)carbamoyl]imino-1,4-dihydro-1-phenylpyridazin;
6-Methoxy-, 6-Ethoxy-, 6-Propoxy- und 6-Butoxy-4-[(sec-butyl)carbamoyl]imino-1,4-dihydro-1-phenylpyridazin;
1,4-Dihydro-4-(isobutylcarbamoyl)imino-1-phenylpyridazin;
6-Methoxy-, 6-Ethoxy-, 6-Propoxy- und 6-Butoxy-1,4-dihydro-4-(isobutylcarbamoyl)imino-1-phenylpyridazin;
4-[(tert-Butyl)carbamoyl]imino-1,4-dihydro-1-phenylpyridazin;
6-Methoxy-, 6-Ethoxy-, 6-Propoxy- und 6-Butoxy-4-[(tert-butyl)carbamoyl]imino-1,4-dihydro-1-phenylpyridazin;
1,4-Dihydro-4-(pentylcarbamoyl)imino-1-phenylpyridazin;
6-Methoxy-, 6-Ethoxy-, 6-Propoxy- und 6-Butoxy-1,4-dihydro-4-(pentylcarbamoyl)imino-1-phenylpyridazin;
1,4-Dihydro-4-(hexylcarbamoyl)imino-1-phenylpyridazin;
6-Methoxy-, 6-Ethoxy-, 6-Propoxy- und Butoxy-1,4-dihydro-4-(hexylcarbamoyl)imino-1-phenylpyridazin;
1,4-Dihydro-4-(heptylcarbamoyl)imino-1-phenylpyridazin;
6-Methoxy-, 6-Ethoxy-, 6-Propoxy- und 6-Butoxy-1,4-dihydro-4-(heptylcarbamoyl)imino-1-phenylpyridazin;
1,4-Dihydro-4-(octylcarbamoyl)imino-1-phenylpyridazin;
6-Methoxy-, 6-Ethoxy-, 6-Propoxy- und 6-Butoxy-1,4-dihydro-4-(octylcarbamoyl)imino-1-phenylpyridazin;
4-(Benzylcarbamoyl)imino-1,4-dihydro-1-phenylpyridazin;
6-Methoxy-, 6-Ethoxy-, 6-Propoxy- und 6-Butoxy-4-(benzylcarbamoyl)imino-1,4-dihydro-1-phenylpyridazin;
1,4-Dihydro-4-(p-methylbenzylcarbamoyl)imino-1-phenylpyridazin;
6-Methoxy-, 6-Ethoxy- und 6-Propoxy-1,4-dihydro-4-(p-methylbenzylcarbamoyl)imino-1-phenylpyridazin;
1,4-Dihydro-4-(m-methoxybenzylcarbamoyl)imino-1-phenylpyridazin;
6-Methoxy-, 6-Ethoxy- und 6-Propoxy-1,4-dihydro-4-(m-methoxybenzylcarbamoyl)imino-1-phenylpyridazin;
1,4-Dihydro-4-(p-methoxybenzylcarbamoyl)imino-1-phenylpyridazin;
6-Methoxy-, 6-Ethoxy- und 6-Propoxy-1,4-dihydro-4-(p-methoxybenzylcarbamoyl)imino-1-phenylpyridazin;
4-(m-Chlorbenzylcarbamoyl)imino-1,4-dihydro-1-phenylpyridazin;
6-Methoxy-, 6-Ethoxy- und 6-Propoxy-4-(m-chlorbenzylcarbamoyl)imino-1,4-dihydro-1-phenylpyridazin;
4-(p-Chlorbenzylcarbamoyl)imino-1,4-dihydro-1-phenylpyridazin;
6-Methoxy-, 6-Ethoxy- und 6-Propoxy-4-(p-chlorbenzylcarbamoyl)imino-1,4-dihydro-1-phenylpyridazin;
1,4-Dihydro-4-(p-fluorbenzylcarbamoyl)imino-1-phenylpyridazin;
6-Methoxy-, 6-Ethoxy- und 6-Propoxy-1,4-dihydro-4-(p-fluorbenzylcarbamoyl)imino-1-phenylpyridazin;
1,4-Dihydro-1-phenyl-4-{[m-(trifluormethyl)benzylcarbamoyl]imino}pyridazin;
6-Methoxy-, 6-Ethoxy- und 6-Propoxy-1,4-dihydro-1-phenyl-4-{[m-(trifluormethyl)benzylcarbamoyl]imino}pyridazin;
1,4-Dihydro-1-phenyl-4-[(phenylcarbamoyl)imino]pyridazin;
6-Methoxy-, 6-Ethoxy-, 6-Propoxy-, 6-Isopropoxy-, 6-Butoxy-1,4-dihydro-1-phenyl-4-[(phenylcarbamoyl)imino]pyridazin;
1,4-Dihydro-1-phenyl-4-[(m-tolylcarbamoyl)imino]pyridazin;
6-Methoxy- und 6-Ethoxy-1,4-dihydro-1-phenyl-4-[(m-tolylcarbamoyl)imino]pyridazin;
1,4-Dihydro-1-phenyl-4-[(p-tolylcarbamoyl)imino]pyridazin;
6-Methoxy- und 6-Ethoxy-1,4-dihydro-1-phenyl-4-[(p-tolylcarbamoyl)imino]pyridazin;
1,4-Dihydro-4-(o-methoxyphenylcarbamoyl)imino-1-phenylpyridazin;
6-Methoxy- und 6-Ethoxy-1,4-dihydro-4-(o-methoxyphenylcarbamoyl)imino-1-phenylpyridazin;
1,4-Dihydro-4-(m-methoxyphenylcarbamoyl)imino-1-phenylpyridazin;
6-Methoxy- und 6-Ethoxy-1,4-dihydro-4-(m-methoxyphenylcarbamoyl)imino-1-phenylpyridazin;
1,4-Dihydro-4-(p-methoxyphenylcarbamoyl)imino-1-phenylpyridazin;
6-Methoxy- und 6-Ethoxy-1,4-dihydro-4-(p-methoxyphenylcarbamoyl)imino-1-phenylpyridazin;
1,4-Dihydro-4-(o,p-dimethoxyphenylcarbamoyl)imino-1-phenylpyridazin;

6-Methoxy- und 6-Ethoxy-1,4-dihydro-4-(o,p-dimethoxyphenylcarbamoyl)imino-1-phenylpyridazin;
1,4-Dihydro-4-[(2,5-dimethoxyphenyl)carbamoyl]imino-1-phenylpyridazin;
6-Methoxy- und 6-Ethoxy-1,4-dihydro-4-[(2,5-dimethoxyphenyl)carbamoyl]imino-1-phenylpyridazin;
4-(o-Chlorphenylcarbamoyl)imino-1,4-dihydro-1-phenylpyridazin;
6-Methoxy- und 6-Ethoxy-4-(o-chlorphenylcarbamoyl)imino-1,4-dihydro-1-phenylpyridazin;
4-(m-Chlorphenylcarbamoyl)imino-1,4-dihydro-1-phenylpyridazin;
6-Methoxy- und 6-Ethoxy-4-(m-chlorphenylcarbamoyl)imino-1,4-dihydro-1-phenylpyridazin;
4-(p-Chlorphenylcarbamoyl)imino-1,4-dihydro-1-phenylpyridazin;
6-Methoxy- und 6-Ethoxy-4-(p-chlorphenylcarbamoyl)imino-1,4-dihydro-1-phenylpyridazin;
4-[(2,3-Dichlorphenyl)carbamoyl]imino-1,4-dihydro-1-phenylpyridazin;
6-Methoxy- und 6-Ethoxy-4-[(2,3-dichlorphenyl)carbamoyl]imino-1,4-dihydro-1-phenylpyridazin;
4-[(3,4-Dichlorphenyl)carbamoyl]imino-1,4-dihydro-1-phenylpyridazin;
6-Methoxy- und 6-Ethoxy-4-[(3,4-dichlorphenyl)carbamoyl]imino-1,4-dihydro-1-phenylpyridazin;
4-(m,m'-Dichlorphenylcarbamoyl)imino-1,4-dihydro-1-phenylpyridazin;
6-Methoxy- und 6-Ethoxy-4-(m,m'-dichlorphenylcarbamoyl)imino-1,4-dihydro-1-phenylpyridazin;
1,4-Dihydro-4-(p-fluorphenylcarbamoyl)imino-1-phenylpyridazin;
6-Methoxy- und 6-Ethoxy-1,4-dihydro-4-(p-fluorphenylcarbamoyl)imino-1-phenylpyridazin;
1,4-Dihydro-1-phenyl-4-{[o-(trifluormethyl)phenylcarbamoyl]imino}pyridazin;
6-Methoxy- und 6-Ethoxy-1,4-dihydro-1-phenyl-4-{[o-(trifluormethyl)phenylcarbamoyl]imino}pyridazin;
1,4-Dihydro-1-phenyl-4-{[m-(trifluormethylcarbamoyl]imino}pyridazin;
6-Methoxy- und 6-Ethoxy-1,4-dihydro-1-phenyl-4-{[m-(trifluormethyl)phenylcarbamoyl]imino}pyridazin;
1,4-Dihydro-4-(methylthiocarbamoyl)imino-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy-, 6-Pentyloxy- und 6-Octyloxy-1,4-dihydro-4-(methylthiocarbamoyl)imino-1-phenylpyridazin;
1,4-Dihydro-4-(ethylthiocarbamoyl)imino-1-phenylpyridazin;
6-Methoxy-, 6-Ethoxy-, 6-Propoxy-, 6-Isopropoxy-, 6-Butoxy- und 6-Pentyloxy-1,4-dihydro-4-(ethylthiocarbamoyl)imino-1-phenylpyridazin;
1,4-Dihydro-1-phenyl-4-[(propylthiocarbamoyl)imino]pyridazin;
6-Methoxy-, 6-Ethoxy-, 6-Propoxy-, 6-Isopropoxy- und 6-Butoxy-1,4-dihydro-1-phenyl-4-[(propylthiocarbamoyl)imino]pyridazin;
1,4-Dihydro-4-(isopropylthiocarbamoyl)imino-1-phenylpyridazin;
6-Methoxy-, 6-Ethoxy-, 6-Propoxy-, 6-Isopropoxy- und 6-Butoxy-1,4-dihydro-4-(isopropylthiocarbamoyl)imino-1-phenylpyridazin;
4-(Butylthiocarbamoyl)imino-1,4-dihydro-1-phenylpyridazin;
6-Methoxy-, 6-Ethoxy-, 6-Propoxy-, 6-Isopropoxy- und 6-Butoxy-4-(butylthiocarbamoyl)imino-1,4-dihydro-1-phenylpyridazin;
4-[(sec-Butyl)thiocarbamoyl]imino-1,4-dihydro-1-phenylpyridazin;
6-Methoxy-, 6-Ethoxy- und 6-Propoxy-4-[(sec-butyl)thiocarbamoyl]imino-1,4-dihydro-1-phenylpyridazin;
1,4-Dihydro-4-(isobutylthiocarbamoyl)imino-1-phenylpyridazin;
6-Methoxy-, 6-Ethoxy- und 6-Propoxy-1,4-dihydro-4-(isobutylthiocarbamoyl)imino-1-phenylpyridazin;
1,4-Dihydro-4-(pentylthiocarbamoyl)imino-1-phenylpyridazin;
6-Methoxy-, 6-Ethoxy-, 6-Propoxy- und 6-Butoxy-1,4-dihydro-4-(pentylthiocarbamoyl)imino-1-phenylpyridazin;
1,4-Dihydro-4-(hexylthiocarbamoyl)imino-1-phenylpyridazin;
6-Methoxy-, 6-Ethoxy-, 6-Propoxy- und 6-Butoxy-1,4-dihydro-4-(hexylthiocarbamoyl)imino-1-phenylpyridazin;
1,4-Dihydro-4-(heptylthiocarbamoyl)imino-1-phenylpyridazin;
6-Methoxy-, 6-Ethoxy-, 6-Propoxy- und 6-Butoxy-1,4-dihydro-4-(heptylthiocarbamoyl)imino-1-phenylpyridazin;
4-(Benzylthiocarbamoyl)imino-1,4-dihydro-1-phenylpyridazin;
6-Methoxy-, 6-Ethoxy- und 6-Propoxy-4-(benzylthiocarbamoyl)imino-1,4-dihydro-1-phenylpyridazin;
1,4-Dihydro-4-(p-methylbenzylthiocarbamoyl)imino-1-phenylpyridazin;
6-Methoxy-, 6-Ethoxy- und 6-Propoxy-1,4-dihydro-4-(p-methylbenzylthiocarbamoyl)imino-1-phenylpyridazin;
1,4-Dihydro-4-(m-methoxybenzylthiocarbamoyl)imino-1-phenylpyridazin;

6-Methoxy-, 6-Ethoxy- und 6-Propoxy-1,4-dihydro-4-(m-methoxybenzylthiocarbamoyl)imino-1-phenylpyridazin;
4-(p-Chlorbenzylthiocarbamoyl)imino-1,4-dihydro-1-phenylpyridazin;
6-Methoxy-, 6-Ethoxy- und 6-Propoxy-4-(p-chlorbenzylthiocarbamoyl)imino-1,4-dihydro-1-phenylpyridazin;
1,4-Dihydro-1-phenyl-4-{[m-(trifluormethyl)benzylthiocarbamoyl]imino}pyridazin;
6-Methoxy-, 6-Ethoxy- und 6-Propoxy-1,4-dihydro-1-phenyl-4-{[m-(trifluormethyl)benzylthiocarbamoyl]imino}pyridazin;
1,4-Dihydro-1-phenyl-4-[(phenylthiocarbamoyl)imino]pyridazin;
6-Methoxy-, 6-Ethoxy- und 6-Propoxy-1,4-dihydro-1-phenyl-4-[(phenylthiocarbamoyl)imino]-pyridazin;
1,4-Dihydro-1-phenyl-4-[(p-tolylthiocarbamoyl)imino]pyridazin;
6-Methoxy-, 6-Ethoxy- und 6-Propoxy-1,4-dihydro-1-phenyl-4-[(p-tolylthiocarbamoyl)imino]pyridazin;
1,4-Dihydro-4-(m-methoxyphenylthiocarbamoyl)imino-1-phenylpyridazin;
6-Methoxy-, 6-Ethoxy- und 6-Propoxy-1,4-dihydro-4-(m-methoxyphenylthiocarbamoyl)imino-1-phenylpyridazin;
4-(p-Chlorphenylthiocarbamoyl)imino-1,4-dihydro-1-phenylpyridazin;
6-Methoxy-, 6-Ethoxy- und 6-Propoxy-4-(p-chlorphenylthiocarbamoyl)imino-1,4-dihydro-1-phenylpyridazin;
1,4-Dihydro-4-(m-fluorphenylthiocarbamoyl)imino-1-phenylpyridazin;
6-Methoxy-, 6-Ethoxy- und 6-Propoxy-1,4-dihydro-4-(m-fluorphenylthiocarbamoyl)imino-1-phenylpyridazin;
1,4-Dihydro-4-(p-fluorphenylthiocarbamoyl)imino-1-phenylpyridazin;
6-Methoxy-, 6-Ethoxy- und 6-Propoxy-1,4-dihydro-4-(p-fluorphenylthiocarbamoyl)imino-1-phenylpyridazin;
1,4-Dihydro-4-(methoxyoxalyl)imino-1-phenylpyridazin;
6-Methoxy-, 6-Ethoxy-, 6-Propoxy-, 6-Isopropoxy-, 6-Butoxy- und 6-Octyloxy-1,4-dihydro-4-(methoxyoxalyl)imino-1-phenylpyridazin;
1,4-Dihydro-4-(ethoxyoxalyl)imino-1-phenylpyridazin;
6-Methoxy-, 6-Ethoxy-, 6-Propoxy-, 6-Isopropoxy-, 6-Butoxy-, 6-(sec-Butoxy)-, 6-Isobutoxy- und 6-Pentyloxy-1,4-dihydro-4-(ethoxyoxalyl)imino-1-phenylpyridazin;
1,4-Dihydro-1-phenyl-4-[(propoxyoxalyl)imino]pyridazin;
6-Methoxy-, 6-Ethoxy-, 6-Propoxy-, 6-Isopropoxy- und 6-Butoxy-1,4-dihydro-1-phenyl-4-[(propoxyoxalyl)imino]pyridazin;
1,4-Dihydro-4-(isopropoxyoxalyl)imino-1-phenylpyridazin;
6-Methoxy-, 6-Ethoxy-, 6-Propoxy- und 6-Butoxy-1,4-dihydro-4-(isopropoxyoxalyl)imino-1-phenylpyridazin;
4-(Butoxyoxalyl)imino-1,4-dihydro-1-phenylpyridazin;
6-Methoxy-, 6-Ethoxy-, 6-Propoxy- und 6-Butoxy-4-(butoxyoxalyl)imino-1,4-dihydro-1-phenylpyridazin;
4-[(sec-Butoxy)oxalyl]imino-1,4-dihydro-1-phenylpyridazin;
6-Methoxy-, 6-Ethoxy-, 6-Propoxy- und 6-Butoxy-4-[(sec-butoxy)oxalyl]imino-1,4-dihydro-1-phenylpyridazin;
1,4-Dihydro-4-(isobutoxyoxalyl)imino-1-phenylpyridazin;
6-Methoxy-, 6-Ethoxy-, 6-Propoxy- und 6-Butoxy-1,4-dihydro-4-(isobutoxyoxalyl)imino-1-phenylpyridazin;
4-[(tert-Butoxy)oxalyl]imino-1,4-dihydro-1-phenylpyridazin;
6-Methoxy-, 6-Ethoxy-, 6-Propoxy- und 6-Butoxy-4-[(tert-butoxy)oxalyl]imino-1,4-dihydro-1-phenylpyridazin;
1,4-Dihydro-1-phenyl-4-[(pentyloxyoxalyl)imino]pyridazin;
6-Methoxy-, 6-Ethoxy-, 6-Propoxy- und 6-Butoxy-1,4-dihydro-1-phenyl-4-[(pentyloxyoxalyl)-imino]pyridazin;
1,4-Dihydro-4-(hexyloxyoxalyl)imino-1-phenylpyridazin;
6-Methoxy-, 6-Ethoxy-, 6-Propoxy- und 6-Butoxy-1,4-dihydro-4-(hexyloxyalyl)imino-1-phenylpyridazin;
1,4-Dihydro-4-(heptyloxyoxalyl)imino-1-phenylpyridazin;
6-Methoxy-, 6-Ethoxy-, 6-Propoxy- und 6-Butoxy-1,4-dihydro-4-(heptyloxyoxalyl)imino-1-phenylpyridazin;
1,4-Dihydro-4-(octyloxyoxalyl)imino-1-phenylpyridazin;
6-Methoxy-, 6-Ethoxy-, 6-Propoxy- und 6-Butoxy-1,4-dihydro-4-(octyloxyoxalyl)imino-1-phenylpyridazin;
und ihre physiologisch verträglichen Säureadditionssalze.

Es wird darauf hingewiesen, daß für die Verbindungen der Formel (I) im Hinblick auf den substituierten Iminorest zwei Formen, die syn- und die anti-Form, in Betracht kommen. Die vorliegende Erfindung betrifft die jeweiligen syn- und anti-Isomeren und deren Gemische.

Bei bestimmten Resten für $R^1$, z. B. (2-Methylcyclopropyl)carbonyl, tritt eine cis/trans-Isomerie auf. Die vorliegende Erfindung betrifft die jeweiligen cis- und trans-Isomeren und deren Gemische. Die reinen cis- oder trans-Verbindungen werden zweckmäßigerweise durch Verwendung reiner Ausgangsverbindungen oder durch Trennung der cis- und trans-Verbindungen durch Kristallisation erhalten.

Die erfindungsgemäßen Verbindungen und ihre physiologisch verträglichen Säureadditionssalze sind als Pharmaka mit antidepressiver oder blutdrucksteigernder Wirkung geeignet bei der Behandlung von psychischen Störungen, insbesondere von Depressionen, und Kreislaufdysregulationen mit hypotonen Zuständen.

Die Untersuchungen der pharmakologischen Eigenschaften wurde nach folgenden Methoden vorgenommen:

Antidepressive Wirkung

Reserpin (2,15 mg/kg subcutan) verursacht an Mäusen (Stamm: Swiss, männlich, Gewicht: 20 bis 26 g) eine Senkung der Körpertemperatur um durchschnittlich 3°C, gemessen 2 h nach Reserpingabe und bei einer Umgebungstemperatur von 20 bis 22°C. Antidepressiva hemmen diese Hypothermie dosisabhängig. Die Applikation der Prüfsubstanzen erfolgt oral 60 min vor der Reserpin-Applikation.

Als ED 50% wird aus der linearen Regression zwischen log Dosis (mg/kg) und relativer Abnahme der Hypothermie ($\Delta$%) die Dosis ermittelt, welche die durch Reserpin ausgelöste Hypothermie um 50% hemmt.

Wirkung auf den Blutdruck

Die blutdrucksteigernde Wirkung wurde an despinalisierten Ratten (Stamm: Sprague Dawley, männlich, Gewicht: 200 bis 280 g) nachgewiesen. Der Blutdruck wurde in der Aorta carotis mit Statham-Druckaufnehmern gemessen. Die Substanzapplikation erfolgte in eine Vena jugularis (wäßrige Lösung, 1 ml/kg) oder intraperitoneal (Traganth-Suspension, 2 ml/kg). Als ED 20% wird die Dosis ermittelt, welche eine 20%ige Blutdrucksteigerung bewirkt.

Die gefundenen Ergebnisse sind in den Tabellen 1 und 2 zusammengestellt, wobei die angegebenen Dosen stets auf Base berechnet sind. Als bekannte und strukturell ähnliche Verbindungen wurden das als Arzneimittel eingeführte Amezinium metilsulfat (1-Phenyl-4-amino-6-methoxy-pyridazinium-methosulfat, DE-C-1 912 941) und die Verbindung Beispiel 1 der DE-A-2 245 248 (1-Phenyl-4-amino-pyridazinium-perchlorat) zum Vergleich herangezogen.

Beide Verbindungen weisen eine antidepressive und eine blutdrucksteigernde Wirkung auf. Der Quotient ED 50% antidepressive Wirkung/ED 20% Blutdrucksteigerung beträgt bei Amezinium 14,7 (bei Prüfung auf Blutdrucksteigerung nach intravenöser Applikation) bzw. 1,98 (bei Prüfung auf Blutdrucksteigerung nach intraperitonealer Applikation), bei der Verbindung des Beispiels 1 der DE-A-2 245 248, 22,2 bzw. 1,6, wie aus Tabelle 1 ersichtlich.

Die erfindungsgemäßen Verbindungen weisen eine ausgeprägte Selektivität auf, entweder ist die antidepressive oder die blutdrucksteigernde Wirkung stärker ausgeprägt, so daß ein besonderes, nicht vorhersehbares Wirkungsprofil in der einen oder anderen Wirkungsrichtung gegeben ist. Erkenntlich ist dies an einem im Verhältnis zu den Vergleichsverbindungen veränderten oben definierten Quotienten. Wird der Quotient kleiner, so ist dies ein Hinweis für eine im Verhältnis zur blutdrucksteigernden Wirkung stärkere und damit selektivere antidepressive Wirkung. Bei Vergrößerung des Quotienten deutet das auf eine im Verhältnis zur antidepressiven Wirkung stärkere und damit selektivere Blutdrucksteigerung hin. Das ergibt sich u. a. daraus, daß der ED 20-Wert der Blutdrucksteigerung im Nenner des Quotienten steht. Die erfindungsgemäße spezielle Substitution an der 4-Iminogruppe ist möglicherweise für die nicht vorhersehbare selektive Wirkungsrichtung verantwortlich.

Bei den Verbindungen der Tabelle 1 ist die antidepressive Wirkung, gemessen an der Hemmung der Reserpin-Hypothermie, selektiver ausgeprägt als bei den Referenzsubstanzen. Der Quotient, antidepressive Wirkung/Blutdrucksteigerung ist mindestens 2fach, maximal 52- bis 77fach kleiner (Beispiele 85, 31) als bei Amezinium.

Die Verbindungen der Tabelle 2 sind besonders blutdrucksteigernd wirksam. Der Blutdruck wird bereits durch Dosen (ED 20%) erhöht, die 24- (Beispiel 14) bis 120mal (Beispiel 30) kleiner sind als die antidepressiv wirksamen Dosen (ED 50%). Bei den Referenzsubstanzen ist die blutdrucksteigernde Dosis nur 15- (Amezinium) bzw. 22mal kleiner.

Tabelle 1

| Beispiel Nr. | Reserpin-Antagonismus Hypothermie Maus, per os ED 50%[1]) | | Blutdrucksteigerung Ratte, despinalisiert i. v. Applikation ED 20%[3]) | | i. p. Applikation ED 20%[3]) | | Q[4]) (i. v.) | (i. p.) |
|---|---|---|---|---|---|---|---|---|
| | mg/kg | R. W.[2]) | mg/kg | R. W. | mg/kg | R. W.[2]) | | |
| 1 | 0,67 | 1,09 | 0,32 | 0,16 | | | 2,09 | |
| 3 | 0,33 | 2,22 | 0,20 | 0,25 | | | 1,65 | |
| 5 | 0,40 | 1,83 | 0,215 | 0,23 | | | 1,82 | |
| 10 | 1,41 | 0,52 | 0,464 | 0,11 | | | 3,04 | |
| 15 | 2,7 | 0,27 | | | >10 | <0,04 | | <0,27 |
| 19 | 1,35 | 0,54 | | | >10 | <0,04 | | <0,14 |
| 20 | 1,1 | 0,67 | | | >10 | <0,04 | | <0,11 |
| 22 | 3,1 | 0,24 | | | 4,64 | 0,08 | | 0,67 |
| 23 | 0,98 | 0,74 | | | 1,0 | 0,37 | | 0,98 |
| 26 | 3,26 | 0,22 | | | 4,64 | 0,08 | | 0,70 |
| 28 | 0,58 | 1,26 | 0,1 | 0,50 | | | 5,80 | |
| 29 | 0,86 | 0,85 | 0,215 | 0,23 | | | 4,00 | |
| 31 | 0,28 | 2,62 | 1,47 | 0,03 | | | 0,19 | |
| 34 | 0,71 | 1,03 | 0,1 | 0,50 | | | 7,10 | |
| 35 | 0,25 | 2,93 | 0,1 | 0,50 | | | 2,50 | |
| 36 | 2,0 | 0,37 | 0,316 | 0,16 | | | 6,33 | |
| 37 | 0,7 | 1,05 | 1,0 | 0,05 | | | 0,70 | |
| 48 | 1,1 | 0,67 | | | 6,81 | 0,05 | | 0,16 |
| 49 | 1,71 | 0,43 | | | 4,64 | 0,08 | | 0,37 |
| 50 | 0,18 | 4,07 | | | 3,16 | 0,12 | | 0,06 |
| 52 | 1,4 | 0,52 | | | 3,16 | 0,12 | | 0,44 |
| 60 | 0,93 | 0,79 | | | 2,15 | 0,17 | | 0,43 |
| 62 | 0,66 | 1,11 | 0,56 | 0,09 | | | 1,18 | |
| 64 | 1,27 | 0,58 | | | >10 | <0,04 | | <0,13 |
| 66 | 1,8 | 0,41 | | | >10 | <0,04 | | <0,18 |
| 68 | 0,95 | 0,77 | | | >21,5 | <0,02 | | <0,04 |
| 69 | 4,86 | 0,15 | 2,15 | 0,02 | | | 2,26 | |

Fortsetzung

| Beispiel Nr. | Reserpin-Antagonismus Hypothermie Maus, per os ED 50%¹) | | Blutdrucksteigerung Ratte, despinalisiert i. v. Applikation ED 20%³) | | i. p. Applikation ED 20%³) | | Q⁴) (i. v.) | (i. p.) |
|---|---|---|---|---|---|---|---|---|
| | mg/kg | R. W.²) | mg/kg | R. W. | mg/kg | R. W.²) | | |
| 71 | 0,76 | 0,96 | 0,464 | 0,11 | | | 1,64 | |
| 72 | 1,47 | 0,50 | 0,464 | 0,11 | | | 3,17 | |
| 73 | 1,23 | 0,60 | | | >10 | <0,04 | | <0,12 |
| 74 | 8,45 | 0,09 | 4,64 | 0,01 | | | 1,82 | |
| 77 | 3,40 | 0,22 | | | 3,16 | 0,12 | | 1,08 |
| 78 | 0,73 | 1,00 | | | 10 | 0,04 | | 0,07 |
| 79 | 1,14 | 0,64 | | | 10 | 0,04 | | 0,11 |
| 80 | 1,5 | 0,49 | | | >10 | <0,04 | | <0,15 |
| 84 | 0,37 | 1,98 | 0,464 | 0,11 | | | 0,80 | |
| 85 | 0,28 | 2,62 | 1,0 | 0,05 | | | 0,28 | |
| 86 | 2,1 | 0,35 | >1,0 | <0,05 | | | <2,1 | |
| 87 | 2,91 | 0,25 | >1,0 | <0,05 | | | <2,91 | |
| Amezinium | 0,733 | 1,00 | 0,05 | 1,00 | 0,37 | 1,00 | 14,66 | 1,98 |
| DE-A-2 245 248 Beispiel 1 | 0,40 | 1,83 | 0,018 | 2,78 | 0,25 | 1,48 | 22,22 | 1,60 |

¹) Dosis, welche die Resperpin-Hypothermie an der Maus um 50% hemmt
²) Relative Wirksamkeit; Amezinium = 1,00
³) Dosis, welche den Blutdruck an der despinalisierten Ratte um 20% steigert
⁴) $Q = \frac{\text{ED 50\% Reserpin-Antagonismus}}{\text{ED 20\% Blutdrucksteigerung}}$

Tabelle 2

| Beispiel Nr. | Blutdrucksteigerung Ratte despinalisiert ED 20%¹) | | Reserpin Antagonismus Hypothermie Maus per os ED 50%³) | | Q⁴) Steigerung |
|---|---|---|---|---|---|
| | mg/kg | R. W.²) | mg/kg | R. W.²) | |
| 8 | 0,055 | 0,91 | 5,23 | 0,14 | 95,09 |
| 9 | 0,063 | 0,79 | 3,98 | 0,18 | 63,17 |
| 11 | 0,077 | 0,65 | 6,09 | 0,12 | 79,09 |
| 12 | 0,022 | 2,27 | 0,75 | 0,98 | 34,09 |

Fortsetzung

| Beispiel Nr. | Blutdrucksteigerung Ratte despinalisiert ED 20%[1]) | | Reserpin Antagonismus Hypothermie Maus per os ED 50%[3]) | | Q[4]) Steigerung |
|---|---|---|---|---|---|
| | mg/kg | R. W.[2]) | mg/kg | R. W.[2]) | |
| 14 | 0,042 | 1,19 | 1,0 | 0,73 | 23,81 |
| 16 | 0,046 | 1,09 | 1,33 | 0,55 | 28,91 |
| 17 | 0,046 | 1,09 | 2,76 | 0,27 | 60,00 |
| 30 | 0,034 | 1,47 | 4,1 | 0,18 | 120,59 |
| 58 | 0,10 | 0,50 | 5,62 | 0,13 | 56,20 |
| 65 | 0,53 | 0,09 | >31,6 | <0,02 | >59,62 |
| Amezinium | 0,05 | 1,00 | 0,733 | 1,00 | 14,66 |
| DE-A-2 245 248 Beispiel 1 | 0,018 | 2,78 | 0,40 | 1,83 | 22,22 |

[1]) Dosis, welche den Blutdruck an der despinalisierten Ratte um 20% steigert
[2]) Relative Wirksamkeit; Amezinium = 1,00
[3]) Dosis, welche die Reserpin-Hypothermie an der Maus um 50% hemmt
[4]) $Q = \frac{\text{ED 50\% Reserpin-Antagonismus}}{\text{ED 20\% Blutdrucksteigerung}}$

Aus den Tabellen ergeben sich als besonders bevorzugt Verbindungen der Formel (I), in der $R^1$ einen Acylrest $-CO-R^3$, in dem $R^3$ für einen Alkylrest mit 1 bis 7 C-Atomen, einen Benzylrest, einen Cycloalkylrest mit 3 bis 6 C-Atomen im Ring, der gegebenenfalls durch einen Methylrest substituiert ist, einen Alkenylrest mit 3 C-Atomen oder einen Phenylrest, der gegebenenfalls ein- oder zweifach durch Fluor, Chlor, Methyl, Methoxy oder Trifluormethyl substituiert ist, steht,
einen Rest $-CO-Y-R^4$, in dem Y für ein Sauerstoffatom und $R^4$ für einen Alkylrest mit 1 bis 4 C-Atomen, einen Benzylrest, einen Phenylrest oder einen Alkenylrest mit 3 C-Atomen stehen, oder einen Rest

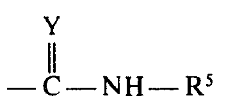

in dem Y ein Schwefelatom bedeutet, wenn für $R^5$ ein Alkylrest mit 1 bis 3 C-Atomen steht, oder Y ein Sauerstoffatom bedeutet, wenn für $R^5$ ein Alkylrest mit 1 bis 4 C-Atomen, ein Benzylrest, ein Phenylrest, der gegebenenfalls einfach durch Fluor, Chlor, Methyl, Methoxy oder Trifluormethyl substituiert ist, steht, und
$R^2$ ein Wasserstoffatom, eine Methoxy- oder Ethoxygruppe bedeuten, und ihre physiologisch verträglichen Säureadditionssalze.

Gegenstand der vorliegenden Erfindung sind demnach auch therapeutische Mittel oder Zubereitungen, die neben pharmazeutisch üblichen Träger- und Verdünnungsmitteln eine Verbindung der Formel (I) oder eines ihrer physiologisch verträglichen Säureadditionssalze als Wirkstoff enthalten.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt (vgl. R. Voigt, Lehrbuch der pharmazeutischen Technologie, VEB-Verlag Volk und Gesundheit, Berlin 1975). Als therapeutische Einzeldosen kommen Dosierungen von 1 bis 500 mg, bevorzugt 5 bis 100 mg, in Betracht.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht.

Weiterhin seien als Zubereitungen beispielsweise auch Suppositorien genannt.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Saccharin, Cyclamat oder Zucker sowie z. B. Aromastoffe, wie Vanillin oder Orangenextrakt, enthalten. Sie können außerdem Suspendierhilfsstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln, wie Neutralfetten oder Polyethylenglykol bzw. deren Derivate herstellen.

Die vorliegende Erfindung wird durch die nachfolgenden Ausführungsbeispiele näher erläutert.

## Beispiel 1

15,0 g (72,2 mmol) 1,4-Dihydro-4-imino-1-phenylpyridazin-hydrochlorid werden unter Rühren in 100 ml Acetanhydrid 2 h auf 100°C erhitzt. Nach dem Abkühlen wird abgesaugt. Der Filterrückstand wird mit Aceton gewaschen und aus Ethanol umkristallisiert. Man isoliert 13,3 g (74% d. Th.) 4-Acetylimino-1,4-dihydro-1-phenylpyridazin-hydrochlorid, farblose Kristalle, Schmelzpunkt 257 bis 260°C.

Analyse für $C_{12}H_{12}ClN_3O$ (249,7):

| | | | | |
|---|---|---|---|---|
| Ber. | C 57,7 | H 4,8 | Cl 14,2 | N 16,8% |
| Gef. | C 57,8 | H 5,0 | Cl 13,9 | N 16,8% |

## Beispiel 2

Zu 8,0 g (29,4 mmol) 1,4-Dihydro-4-imino-1-phenylpyridazin-perchlorat in 100 ml Acetanhydrid gibt man 2 Tropfen konzentrierter Schwefelsäure. Das Gemisch wird anschließend auf Rückflußtemperatur gebracht und für kurze Zeit bei dieser Temperatur gehalten. Nach dem Abkühlen wird abgesaugt. Als Filterrückstand verbleiben 7,0 g (76% d. Th.) 4-Acetylimino-1,4-dihydro-1-phenylpyridazin-perchlorat, Schmelzpunkt 229 bis 231 °C (Zersetzung) (umkristallisiert aus Acetonitril).

Analyse für $C_{12}H_{12}ClN_3O_5$ (313,7):

| | | | | |
|---|---|---|---|---|
| Ber. | C 45,9 | H 3,9 | Cl 11,3 | N 13,4% |
| Gef. | C 46,1 | H 3,8 | Cl 11,7 | N 13,7% |

## Beispiel 3

5,0 g (24,1 mmol) 1,4-Dihydro-4-imino-1-phenylpyridazin-hydrochlorid werden in 50 ml Propionylchlorid 20 h unter Rühren bei Rückflußtemperatur gehalten. Nach dem Abkühlen wird abgesaugt und der Filterrückstand aus Ethanol/Methyltertiärbutylether umkristallisiert. Ausbeute: 2,6 g (41% d. Th.) 1,4-Dihydro-1-phenyl-4-(propionylimino)pyridazin-hydrochlorid, leicht gelbliche Kristalle, Schmelzpunkt 214 bis 215°C.

Analyse für $C_{13}H_{14}ClN_3O$ (263,7):

| | | | | |
|---|---|---|---|---|
| Ber. | C 59,2 | H 5,4 | Cl 13,4 | N 15,9% |
| Gef. | C 59,1 | H 5,3 | Cl 13,3 | N 15,8% |

Beispiel 4

5,0 g (24,1 mmol) 1,4-Dihydro-4-imino-1-phenylpyridazin-hydrochlorid werden in 50 ml Butyrylchlorid 15 h unter Rühren bei Rückflußtemperatur gehalten. Nach dem Abkühlen wird abgesaugt. Der Filterrückstand wird mit Aceton gewaschen und aus Isopropanol umkristallisiert. Man isoliert 4,8 g (72% d. Th.) 4-Butyrylimino-1,4-dihydro-1-phenylpyridazin-hydrochlorid, hellbeige Kristalle, Schmelzpunkt 205 bis 208°C.

Analyse für $C_{14}H_{16}ClN_3O$ (277,8):

| | | | | |
|---|---|---|---|---|
| Ber. | C 60,5 | H 5,8 | Cl 12,8 | N 15,1% |
| Gef. | C 60,4 | H 5,8 | Cl 13,0 | N 15,2% |

Beispiel 5

5,0 g (21,4 mmol) 1,4-Dihydro-4-imino-1-phenylpyridazin-hydrochlorid werden in 20 ml Isobutyrylchlorid 20 h unter Rühren bei Rückflußtemperatur gehalten. Nach dem Abkühlen wird abgesaugt. Der Filterrückstand wird mit Aceton gewaschen und aus Ethanol/Methyltertiärbutylether umkristallisiert. Ausbeute: 4,5 g (67% d. Th.) 1,4-Dihydro-4-isobutyrylimino-1-phenylpyridazin-hydrochlorid, farblose Kristalle, Schmelzpunkt 240 bis 242°C.

Analyse für $C_{14}H_{16}ClN_3O$ (277,8):

| | | | | |
|---|---|---|---|---|
| Ber. | C 60,5 | H 5,8 | Cl 12,8 | N 15,1% |
| Gef. | C 60,6 | H 5,9 | Cl 13,0 | N 15,2% |

Beispiel 6

Zu 9,3 g (44,8 mmol) 1,4-Dihydro-4-imino-1-phenylpyridazin-hydrochlorid und 5,3 g (49,7 mmol) Isobutyrylchlorid in 50 ml Methylenchlorid werden bei Raumtemperatur unter Rühren 11,5 g (114 mmol) Triethylamin getropft. Nach beendeter Zugabe wird noch 4 h bei Raumtemperatur gerührt. Nun wird das Reaktionsgemisch mit Wasser versetzt. Die organische Phase wird abgetrennt und mit verdünnter Salzsäure (100 ml Wasser und 20 ml konz. Salzsäure) gut durchgemischt. Die Methylenchlorid-Phase wird verworfen. Die wäßrige Schicht wird mit Natronlauge auf pH 10 eingestellt. Die hierbei gebildete Festsubstanz wird abgesaugt, mit Wasser gewaschen, getrocknet und aus Toluol/Hexan umkristallisiert. Man erhält 5,1 g (47% d. Th.) 1,4-Dihydro-4-isobutyrylimino-1-phenylpyridazin, gelbbeige Kristalle, Schmelzpunkt 138°C.

Analyse für $C_{14}H_{15}N_3O$ (241,3):

| | | | |
|---|---|---|---|
| Ber. | C 69,7 | H 6,3 | N 17,4% |
| Gef. | C 69,6 | H 6,2 | N 17,7% |

Beispiel 7

6,0 g (28,9 mmol) 1,4-Dihydro-4-imino-1-phenylpyridazin-hydrochlorid werden in 40 ml Valerylchlorid 20 h unter Rühren bei 120°C gehalten. Nach dem Abkühlen wird abgesaugt und der Filterrückstand mit Aceton gewaschen. Umkristallisiert wird zuerst aus Etanol/Methyltertiärbutylether und dann noch aus Ethanol/Aceton. Man erhält 3,4 g (40% d. Th.) 1,4-Dihydro-1-phenyl-4-(valerylimino)pyridazin-hydrochlorid, leicht gelbliche Kristalle, Schmelzpunkt 180 bis 182°C.

Analyse für $C_{15}H_{18}ClN_3O$ (291,8):

| | | | | |
|---|---|---|---|---|
| Ber. | C 61,7 | H 6,2 | Cl 12,2 | N 14,4% |
| Gef. | C 61,8 | H 6,2 | Cl 11,9 | N 14,5% |

Beispiel 8

Führt man das Beispiel 7 unter Verwendung von Octanoylchlorid an Stelle von Valerylchlorid durch, so erhält man nach der Umkristallisation aus Ethanol/Methyltertiärbutylether 2,9 g (30% d. Th.) 1,4-Dihydro-4-octanoylimino-1-phenylpyridazin-hydrochlorid, farblose Kristalle, Schmelzpunkt 167

bis 169°C.

Analyse für $C_{18}H_{24}ClN_3O$ (333,9):

| | | | | |
|---|---|---|---|---|
| Ber. | C 64,8 | H 7,2 | Cl 10,6 | N 12,6% |
| Gef. | C 64,9 | H 7,2 | Cl 11,0 | N 12,6% |

Beispiel 9

6,0 g (28,9 mmol) 1,4-Dihydro-4-imino-1-phenylpyridazin-hydrochlorid werden in 50 ml Phenylacetylchlorid 6 h unter Rühren bei 120°C gehalten. Nach dem Abkühlen wird eingeengt und der Rückstand mit Methanol/Methyltertiärbutylether behandelt. Man saugt ab und kristallisiert den Feststoff zuerst aus Ethanol und dann noch aus Ethanol/Aceton unter Zusatz von Aktivkohle um. Ausbeute: 3,9 g (41% d. Th.) 1,4-Dihydro-1-phenyl-4-[(phenylacetyl)imino]pyridazin-hydrochlorid, hellbeige Kristalle, Schmelzpunkt 208 bis 210°C.

Analyse für $C_{18}H_{16}ClN_3O$ (325,8):

| | | | | |
|---|---|---|---|---|
| Ber. | C 66,4 | H 5,0 | Cl 10,9 | N 12,9% |
| Gef. | C 66,5 | H 5,0 | Cl 11,2 | N 13,0% |

Beispiel 10

6,0 g (28,9 mmol) 1,4-Dihydro-4-imino-1-phenylpyridazin-hydrochlorid werden in 35 ml Cyclopropancarbonsäurechlorid 20 h unter Rühren bei Rückflußtemperatur gehalten. Nach dem Abkühlen wird abgesaugt. Der Filterrückstand wird mit Aceton und aus Isopropanol umkristallisiert. Man erhält 5,1 g (64% d. Th.) 4-(Cyclopropylcarbonyl)imino-1,4-dihydro-1-phenylpyridazin-hydrochlorid, hellbeige Kristalle, Schmelzpunkt 245 bis 247°C.

Analyse für $C_{14}H_{14}ClN_3O$ (275,7):

| | | | | |
|---|---|---|---|---|
| Ber. | C 61,0 | H 5,1 | Cl 12,9 | N 15,2% |
| Gef. | C 61,0 | H 5,2 | Cl 13,2 | N 15,0% |

Beispiel 11

6,0 g (28,9 mmol) 1,4-Dihydro-4-imino-1-phenylpyridazin-hydrochlorid und 15 ml Cyclobutancarbonsäurechlorid werden in 10 ml Toluol unter Rühren 16 h bei Rückflußtemperatur gehalten. Nach dem Abkühlen wird abgesaugt. Der Filterrückstand wird mit Aceton gewaschen und aus Ethanol umkristallisiert. Man isoliert 4,2 g (50% d. Th.) 4-(Cyclobutylcarbonyl)imino-1,4-dihydro-1-phenylpyridazin-hydrochlorid, farblose Kristalle, Schmelzpunkt 232 bis 235°C.

Analyse für $C_{15}H_{16}ClN_3O$ (289,8):

| | | | | |
|---|---|---|---|---|
| Ber. | C 62,2 | H 5,6 | Cl 12,2 | N 14,5% |
| Gef. | C 62,4 | H 5,5 | Cl 12,2 | N 14,5% |

Beispiel 12

Zu 15,0 g (72,2 mmol) 1,4-Dihydro-4-imino-1-phenylpyridazin-hydrochlorid und 12,8 g (~98 mmol) ~80%iges Methacrylsäurechlorid in 200 ml Methylenchlorid werden bei Raumtemperatur unter Rühren 24,0 g (237 mmol) Triethylamin getropft. Nach beendeter Zugabe wird noch 2 h bei Raumtemperatur nachgerührt. Nun wird das Reaktionsgemisch mit Wasser versetzt. Die organische Phase wird abgetrennt, mit Wasser gewaschen und eingeengt. Den Rückstand kristallisiert man dreimal aus Toluol um. Ausbeute: 3,6 g (21% d. Th.) 1,4-Dihydro-4-methacryloylimino-1-phenylpyridazin, gelbe Kristalle, Schmelzpunkt 136°C.

Analyse für $C_{14}H_{13}N_3O$ (239,3):

| | | | |
|---|---|---|---|
| Ber. | C 70,3 | H 5,5 | N 17,6% |
| Gef. | C 70,4 | H 5,5 | N 17,6% |

Beispiel 13

6,0 g (28,9 mmol) 1,4-Dihydro-4-imino-1-phenylpyridazin-hydrochlorid werden in 50 ml Benzoylchlorid 10 h unter Rühren bei 120°C gehalten. Nach dem Abkühlen wird abgesaugt. Der Filterrückstand wird mit Aceton gewaschen und aus Isopropanol umkristallisiert. Man erhält 3,3 g (37% d. Th.) 4-Benzoylimino-1,4-dihydro-1-phenylpyridazin-hydrochlorid, hellbeige Kristalle, Schmelzpunkt 225 bis 226°C (Zersetzung).

Analyse für $C_{17}H_{14}ClN_3O$ (311,8):

| | | | | |
|---|---|---|---|---|
| Ber. | C 65,5 | H 4,5 | Cl 11,4 | N 13,5% |
| Gef. | C 65,6 | H 4,5 | Cl 11,0 | N 13,5% |

Beispiel 14

10,0 g (48,2 mmol) 1,4-Dihydro-4-imino-1-phenylpyridazin-hydrochlorid und 20 ml (26,8 g (169 mmol)) 4-Fluorbenzoylchlorid werden in 30 ml Toluol 5 h unter Rühren bei 120°C gehalten. Nach dem Abkühlen wird abgesaugt und aus Ethanol umkristallisiert. Man erhält 6,2 g (39% d. Th.) 1,4-Dihydro-4-(4-fluorbenzoyl)imino-1-phenylpyridazin-hydrochlorid, hellbeige, Kristalle, Schmelzpunkt 257 bis 258°C.

Analyse für $C_{17}H_{13}ClFN_3O$ (329,8):

| | | | | | |
|---|---|---|---|---|---|
| Ber. | C 61,9 | H 4,0 | Cl 10,8 | F 5,8 | N 12,7% |
| Gef. | C 62,0 | H 4,0 | Cl 10,8 | F 5,9 | N 12,9% |

Beispiel 15

Zu 7,5 g (36,1 mmol) 1,4-Dihydro-4-imino-1-phenylpyridazin-hydrochlorid und 8,3 g (39,6 mmol) 3,5-Dichlorbenzoylchlorid in 100 ml Methylenchlorid werden bei Raumtemperatur unter Rühren 9,6 g (94,9 mmol) Triethylamin getropft. Anschließend wird noch 2 h bei Raumtemperatur nachgerührt. Man saugt ab, wäscht zuerst mit Wasser, dann mit Aceton und kristallisiert aus Toluol um. Ausbeute: 8,0 g (64% d. Th.) 4-(3,5-Dichlorbenzoyl)imino-1,4-dihydro-1-phenylpyridazin, gelbe Kristalle, Schmelzpunkt 242 bis 244°C.

Analyse für $C_{17}H_{11}Cl_2N_3O$ (344,2):

| | | | | |
|---|---|---|---|---|
| Ber. | C 59,3 | H 3,2 | Cl 20,6 | N 12,2% |
| Gef. | C 59,5 | H 3,3 | Cl 20,7 | N 12,3% |

Beispiel 16

Zu der Lösung von 10,0 g (48,2 mmol) 1,4-Dihydro-4-imino-1-phenylpyridazin-hydrochlorid in 80 ml Wasser werden 80 ml Toluol gegeben. Das Zwei-Phasensystem wird bei Raumtemperatur unter Rühren zuerst mit 6,8 g (48,4 mmol) Benzoylchlorid und anschließend innerhalb 15 min mit 4,0 g (100 mmol) NaOH in 20 ml Wasser versetzt. Es wird noch 1 h bei Raumtemperatur gerührt und dann abgesaugt. Die erhaltene Festsubstanz wird mit Wasser gewaschen, getrocknet und aus Ethanol umkristallisiert. Man isoliert 9,5 g (72% d. Th.) 4-Benzoylimino-1,4-dihydro-1-phenylpyridazin, gelbe Kristalle, Schmelzpunkt 175 bis 176°C.

Analyse für $C_{17}H_{13}N_3O$ (275,3):

| | | | | |
|---|---|---|---|---|
| Ber. | C 74,2 | H 4,8 | N 15,3 | O 5,8% |
| Gef. | C 74,0 | H 4,8 | N 15,4 | O 5,9% |

In der folgenden Tabelle (Tabelle 1) sind die Beispiele 17 bis 28 zusammengestellt. Die Herstellung dieser Pyridazinonimine erfolgt nach der im Beispiel 16 beschriebenen Methode.

Tabelle 1

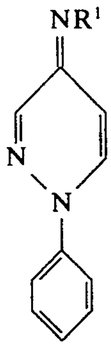

| Bei-spiel | $R^1$ | Schmelzpunkt [°C] | Aus-beute (%) | | Analyse (%) C | H | Cl/(F) | N | O |
|---|---|---|---|---|---|---|---|---|---|
| 17 | 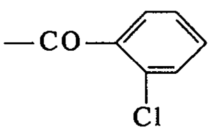 | 136–138 | 60 | Ber. | 65,9 | 3,9 | 11,4 | 13,6 | 5,2 |
| | | (Ethanol/Toluol) | | Gef. | 65,6 | 4,1 | 12,1 | 13,5 | 5,1 |
| 18 | 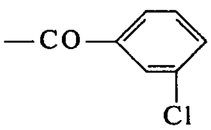 | 212–214 | 61 | Ber. | 65,9 | 3,9 | 11,4 | 13,6 | 5,2 |
| | | (Toluol) | | Gef. | 65,9 | 3,9 | 11,3 | 13,7 | 5,2 |
| 19 | 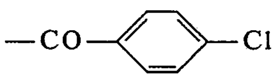 | 216 | 60 | Ber. | 65,9 | 3,9 | 11,4 | 13,6 | 5,2 |
| | | (Toluol) | | Gef. | 65,9 | 4,0 | 11,6 | 13,6 | 5,2 |
| 20 | 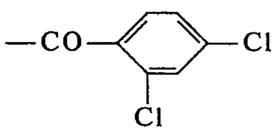 | 159–160 | 50 | Ber. | 59,3 | 3,2 | 20,6 | 12,2 | |
| | | (Toluol) | | Gef. | 59,6 | 3,2 | 20,7 | 12,5 | |
| 21 | 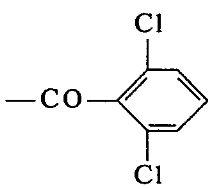 | 163–165 | 27 | Ber. | 59,3 | 3,2 | 20,6 | 12,2 | 4,6 |
| | | (Toluol) | | Gef. | 59,4 | 3,3 | 20,5 | 12,3 | 4,6 |
| 22 | 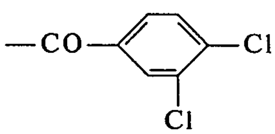 | 159–160 | 40 | Ber. | 59,3 | 3,2 | 20,6 | 12,2 | 4,6 |
| | | (Toluol) | | Gef. | 59,5 | 3,3 | 20,4 | 12,2 | 4,8 |
| 23 | 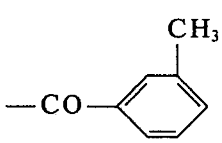 | 158–159 | 54 | Ber. | 74,7 | 5,2 | – | 14,5 | |
| | | (Toluol) | | Gef. | 74,8 | 5,3 | – | 14,7 | |
| 24 | 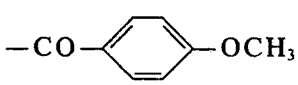 | 153–154 | 05 | Ber. | 70,8 | 5,0 | – | 13,8 | |
| | | (Toluol) | | Gef. | 70,8 | 5,0 | – | 13,9 | |

Fortsetzung

| Beispiel | $R^1$ | Schmelzpunkt [°C] | Ausbeute (%) | Analyse (%) | C | H | Cl/(F) | N | O |
|---|---|---|---|---|---|---|---|---|---|
| 25 | 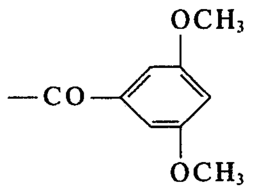 | 155 (Toluol) | 30 | Ber.<br>Gef. | 68,0<br>68,3 | 5,1<br>5,1 | -<br>- | 12,5<br>12,7 | |
| 26 | 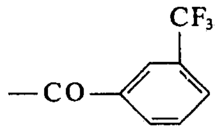 | 163 (Isobutanol) | 65 | Ber.<br>Gef. | 63,0<br>63,0 | 3,5<br>3,6 | (16,6)<br>(16,9) | 12,2<br>12,5 | |
| 27 | 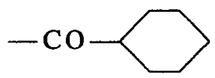 | 159 (Toluol/ Cyclohexanon) | 46 | Ber.<br>Gef. | 72,6<br>72,5 | 6,8<br>6,8 | -<br>- | 14,9<br>15,0 | |
| 28 | 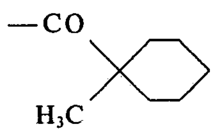 | 158 (Toluol/ Cyclohexanon) | 39 | Ber.<br>Gef. | 73,2<br>73,3 | 7,2<br>7,1 | -<br>- | 14,2<br>14,2 | |

## Beispiel 29

6,0 g (28,9 mmol) 1,4-Dihydro-4-imino-1-phenylpyridazin-hydrochlorid werden in 45 ml Chlorameisensäureethylester 4 Tage unter Rühren bei Rückflußtemperatur gehalten. Nach dem Abkühlen wird abgesaugt und der Filterrückstand über eine $SiO_2$-Säule chromatographiert (Chloroform/Methanol/Ameisensäure 90 : 9,5 : 0,5 Vol.-%). Das eluierte 1,4-Dihydro-4-(ethoxycarbonyl)imino-1-phenylpyridazin-hydrochlorid wird aus Methanol/Ether umkristallisiert. Ausbeute: 2,3 g (28% d. Th.) leicht rötliche Kristalle vom Schmelzpunkt 161 bis 162°C (Zersetzung).

Analyse für $C_{13}H_{14}ClN_3O_2$ (279,7):

| | | | | |
|---|---|---|---|---|
| Ber. | C 55,8 | H 5,0 | Cl 12,7 | N 15,0% |
| Gef. | C 55,8 | H 5,1 | Cl 12,6 | N 15,1% |

## Beispiel 30

6,0 g (28,9 mmol) 1,4-Dihydro-4-imino-1-phenylpyridazin-hydrochlorid werden in 40 ml Chlorameisensäurephenylester 4 Tage unter Rühren bei 95°C gehalten. Nach dem Abkühlen wird abgesaugt. Der Filterrückstand wird zuerst aus Ethanol/Methyltertiärbutylether und dann noch aus Dimethylformamid/Essigester umkristallisiert. Man isoliert 3,3 g (35% d. Th.) 1,4-Dihydro-4-(phenoxycarbonyl)imino-1-phenylpyridazin-hydrochlorid, hellorange Kristalle, Schmelzpunkt 222 bis 223°C.

Analyse für $C_{17}H_{14}ClN_3O_2$ (327,8):

| | | | | |
|---|---|---|---|---|
| Ber. | C 62,3 | H 4,3 | Cl 10,8 | N 12,8% |
| Gef. | C 62,3 | H 4,4 | Cl 10,9 | N 13,1% |

## Beispiel 31

Zu 10,0 g (48,2 mmol) 1,4-Dihydro-4-imino-1-phenylpyridazin-hydrochlorid in 25 ml (24,2 g (424 mmol)) Methylisocyanat werden bei Raumtemperatur unter Rühren 5,3 g (52,4 mmol) Triethylamin getropft. Nach beendeter Zugabe wird das Reaktionsgemisch mit 50 ml Aceton verdünnt und noch 2 h bei Raumtemperatur gerührt. Es wird abgesaugt, mit Wasser und Aceton nachgewaschen und aus Methanol umkristallisiert. Ausbeute: 3,0 g (27% d. Th.) 1,4-Dihydro-4-(methylcarbamoyl)imino-1-phenylpyridazin, leicht grünliche Kristalle, Schmelzpunkt 205 bis 206°C.

Analyse für $C_{12}H_{12}N_4O$ (228,3):

| | | | |
|---|---|---|---|
| Ber. | C 63,1 | H 5,3 | N 24,5% |
| Gef. | C 63,1 | H 5,2 | N 24,7% |

## Beispiel 32

Zu 10,0 g (48,2 mmol) 1,4-Dihydro-4-imino-1-phenylpyridazin-hydrochlorid in 20 ml (22,6 g (167 mmol)) Phenylisothiocyanat werden bei Raumtemperatur unter Rühren 5,3 g (52,4 mmol) Triethylamin getropft. Nach beendeter Zugabe wird das Reaktionsgemisch noch 2 h bei Raumtemperatur gerührt und dann mit 30 ml Aceton verdünnt. Es wird abgesaugt und der Filterrückstand mit Aceton, Wasser und nochmals Aceton gewaschen und aus Ethanol umkristallisiert. Man isoliert 6,0 g (41% d. Th.) 1,4-Dihydro-1-phenyl-4-[(phenylthiocarbamoyl)imino]pyridazin, orangenfarbene Kristalle, Schmelzpunkt 171 bis 173°C.

Analyse für $C_{17}H_{14}N_4S$ (306,4):

| | | | | |
|---|---|---|---|---|
| Ber. | C 66,6 | H 4,6 | N 18,3 | S 10,5% |
| Gef. | C 66,7 | H 4,6 | N 18,4 | S 10,3% |

In der folgenden Tabelle (Tabelle 2) sind die Beispiele 33 bis 46 zusammengestellt. Die Synthese dieser Pyridazinonimine erfolgt nach der im Beispiel 16 beschriebenen Methode. Als Reagenz wird anstatt des dort verwendeten Benzoylchlorids ein Chlorameisensäureester (Beispiele 33 bis 35), Chlorthioameisensäure-S-ethylester (Beispiel 36), ein Isocyanat (Beispiele 37 bis 44) oder ein Isothiocyanat (Beispiele 45 und 46) eingesetzt. Bei Einsatz eines Isocyanats oder Isothiocyanats verwendet man auf 48,2 mmol Substrat nur 50 mmol NaOH.

Tabelle 2

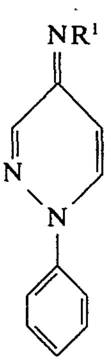

| Beispiel | $R^1$ | Schmelzpunkt [°C] | Ausbeute (%) | Analyse (%) | C | H | N | S |
|---|---|---|---|---|---|---|---|---|
| 33 | $—CO—O—(CH_2)_3—CH_3$ | 110-119 (Toluol/Cyclohexan) | 32 | Ber.<br>Gef. | 66,4<br>66,8 | 6,3<br>6,3 | 15,5<br>15,5 | –<br>– |
| 34 | $—CO—O—CH_2—$ 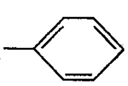 | 115 (Dimethylformamid/ Wasser) | 24 | Ber.<br>Gef. | 70,8<br>70,7 | 5,0<br>5,0 | 13,8<br>13,7 | –<br>– |
| 35 | $—CO—O—CH_2—CH{=}CH_2$ | 123 (Toluol) | 61 | Ber.<br>Gef. | 65,9<br>66,1 | 5,1<br>5,1 | 16,5<br>16,5 | –<br>– |

Tabelle 2 (Fortsetzung)

| Beispiel | $R^1$ | Schmelzpunkt [°C] | Ausbeute (%) | | Analyse (%) C | H | N | S |
|---|---|---|---|---|---|---|---|---|
| 36 | $—CO—S—CH_2—CH_3$ | 120-121 (Toluol) | 26 | Ber.<br>Gef. | 60,2<br>60,1 | 5,1<br>5,0 | 16,2<br>16,3 | 12,4<br>12,2 |
| 37 | $—CO—NH—CH(CH_3)_2$ | 137-138 (Methanol) | 31 | Ber.<br>Gef. | 65,6<br>65,4 | 6,3<br>6,4 | 21,9<br>22,0 | -<br>- |

Tabelle 2 (Fortsetzung)

| Beispiel | $R^1$ | Schmelzpunkt [°C] | Ausbeute (%) | | Analyse (%) C | H | Cl | N |
|---|---|---|---|---|---|---|---|---|
| 38 | $—CO—NH—(CH_2)_3—CH_3$ | 142-143 (Toluol) | 61 | Ber.<br>Gef. | 66,6<br>66,7 | 6,7<br>6,6 | -<br>- | 20,7<br>20,7 |
| 39 | 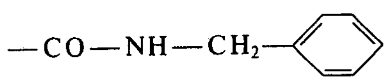 | 177 (Ethanol) | 71 | Ber.<br>Gef. | 71,0<br>71,0 | 5,3<br>5,4 | -<br>- | 18,4<br>18,6 |
| 40 | 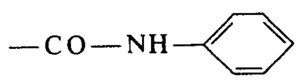 | 164 (Toluol) | 50 | Ber.<br>Gef. | 70,3<br>70,5 | 4,9<br>4,9 | -<br>- | 19,3<br>19,3 |
| 41 | 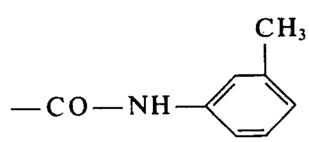 | 129-131 (Toluol) | 42 | Ber.<br>Gef. | 71,0<br>71,0 | 5,3<br>5,3 | -<br>- | 18,4<br>18,7 |
| 42 | 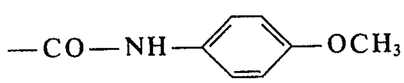 | 150-151 (Toluol) | 43 | Ber.<br>Gef. | 67,5<br>67,6 | 5,0<br>5,0 | -<br>- | 17,5<br>17,6 |
| 43 | 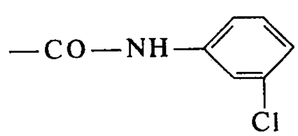 | 152-153 (Ethanol) | 62 | Ber.<br>Gef. | 62,9<br>62,9 | 4,0<br>3,9 | 10,9<br>11,0 | 17,3<br>17,4 |

Tabelle 2 (Fortsetzung)

| Beispiel | $R^1$ | Schmelzpunkt [°C] | Ausbeute (%) | | Analyse (%) C | H | Cl/(F) | N | S |
|---|---|---|---|---|---|---|---|---|---|
| 44 | 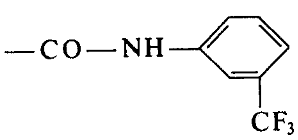 | 167 (Butanol) | 36 | Ber.<br>Gef. | 60,3<br>60,5 | 3,7<br>3,7 | (15,9)<br>(15,6) | 15,6<br>15,7 | -<br>- |
| 45 | 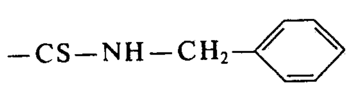 | 142-143 (Methylenchlorid/ Hexan) | 58 | Ber.<br>Gef. | 67,5<br>67,4 | 5,0<br>5,1 | -<br>- | 17,5<br>17,6 | 10,0<br>9,8 |
| 46 | 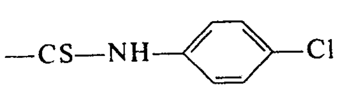 | 156-159 (Ethanol) | 42 | Ber.<br>Gef. | 59,9<br>59,9 | 3,8<br>4,1 | 10,4<br>10,7 | 16,4<br>16,2 | 9,4<br>9,4 |

## Beispiel 47

10,0 g (48,2 mmol) 1,4-Dihydro-4-imino-1-phenylpyridazin-hydrochlorid und 10 ml (12,2 g (89,5 mmol)) Oxalsäureethylesterchlorid werden in 50 ml Toluol unter Rühren 4 h bei Rückflußtemperatur gehalten. Nach dem Abkühlen wird abgesaugt. Der Filterrückstand wird mit Aceton gewaschen und aus Ethanol umkristallisiert. Man isoliert 6,5 g (44% d. Th.) 1,4-Dihydro-4-(ethoxyoxalyl)imino-1-phenylpyridazin-hydrochlorid, leicht gelbliche Kristalle, Schmelzpunkt 169 bis 170°C.

Analyse für $C_{14}H_{14}ClN_3O_3$ (307,7):

| | | | | |
|---|---|---|---|---|
| Ber. | C 54,6 | H 4,6 | Cl 11,5 | N 13,7% |
| Gef. | C 54,6 | H 4,5 | Cl 11,5 | N 13,6% |

## Beispiel 48

Zu 15,5 g (49,5 mmol) 1,4-Dihydro-4-imino-6-methoxy-1-phenylpyridazin-methylsulfat in 100 ml Acetanhydrid gibt man einige Tropfen konz. Schwefelsäure und erhitzt anschließend unter Rühren auf 85°C bis eine klare Lösung entsteht. Nun wird unter vermindertem Druck eingeengt und der Rückstand in ca. 100 ml Wasser gelöst. Die Lösung wird mit 10 g 70%iger Perchlorsäure versetzt. Der dabei gebildete Niederschlag wird abgesaugt und unter vermindertem Druck getrocknet. Ausbeute: 15,0 g (88% d. Th.) 4-Acetylimino-1,4-dihydro-6-methoxy-1-phenylpyridazin-perchlorat vom Schmelzpunkt 205 bis 207°C (Zersetzung).

Analyse für $C_{13}H_{14}ClN_3O_6$ (343,7):

| | | | | |
|---|---|---|---|---|
| Ber. | C 45,4 | H 4,1 | Cl 10,3 | N 12,2% |
| Gef. | C 46,4 | H 4,4 | Cl 10,3 | N 12,4% |

In der folgenden Tabelle (Tabelle 3) sind die Beispiele 49 bis 57 zusammengestellt. Die Herstellung dieser Verbindungen erfolgte nach der im Beispiel 48 beschriebenen Methode (für die Beispiele 49, 51, 53, 55 und 57 wurde als Substrat anstatt 1,4-Dihydro-4-imino-6-methoxy-1-phenylpyridazin-methylsulfat 1,4-Dihydro-6-ethoxy-4-imino-1-phenylpyridazin-ethylsulfat eingesetzt).

Tabelle 3

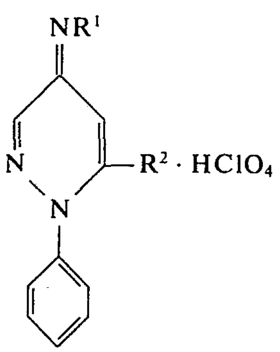

| Beispiel | $R^1$ | $R^2$ | Schmelzpunkt [°C] | Ausbeute (%) | Analyse (%) | C | H | Cl | N |
|---|---|---|---|---|---|---|---|---|---|
| 49 | $-CO-CH_3$ | $-OC_2H_5$ | 131-136 | 66 | Ber. | 47,0 | 4,5 | 9,9 | 11,7 |
| | | | | | Gef. | 46,8 | 4,5 | 10,1 | 11,8 |
| 50 | $-CO-CH_2-CH_3$ | $-OCH_3$ | 130-140 | 49 | Ber. | 47,0 | 4,5 | 9,9 | 11,7 |
| | | | | | Gef. | 47,1 | 4,6 | 9,4 | 11,8 |
| 51 | $-CO-CH_2-CH_3$ | $-OC_2H_5$ | 173-174 | 60 | Ber. | 48,5 | 4,9 | 9,5 | 11,3 |
| | | | | | Gef. | 48,5 | 4,9 | 9,5 | 11,5 |

Tabelle 3 (Fortsetzung)

| Beispiel | $R^1$ | $R^2$ | Schmelzpunkt [°C] | Ausbeute (%) | | Analyse (%) C | H | Cl | N |
|---|---|---|---|---|---|---|---|---|---|
| 52 | $-CO-(CH_2)_2-CH_3$ | $-OCH_3$ | 141-142 | 33 | Ber. | 48,5 | 4,9 | 9,5 | 11,3 |
| | | | | | Gef. | 46,6 | 5,0 | 9,5 | 11,3 |
| 53 | $-CO-(CH_2)_2-CH_3$ | $-OC_2H_5$ | 209-211 | 52 | Ber. | 49,8 | 5,2 | 9,2 | 10,9 |
| | | | | | Gef. | 50,1 | 5,4 | 9,1 | 11,2 |
| 54 | $-CO-(CH_2)_4-CH_3$ | $-OCH_3$ | 149-150 | 20 | Ber. | 51,1 | 5,5 | 8,9 | 10,5 |
| | | | | | Gef. | 51,1 | 5,6 | 9,2 | 10,5 |
| 55 | $-CO-(CH_2)_4-CH_3$ | $-OC_2H_5$ | 117-121 | 22 | Ber. | 52,2 | 5,9 | 8,6 | 10,2 |
| | | | | | Gef. | 52,4 | 5,9 | 8,5 | 10,0 |
| 56 | $-CO-(CH_2)_5-CH_3$ | $-OCH_3$ | 152-153 | 27 | Ber. | 52,2 | 5,9 | 8,6 | 10,2 |
| | | | | | Gef. | 52,4 | 5,9 | 8,8 | 10,1 |
| 57 | $-CO-(CH_2)_5-CH_3$ | $-OC_2H_5$ | 73-74 | 49 | Ber. | 53,3 | 6,1 | 8,3 | 9,8 |
| | | | | | Gef. | 53,7 | 6,2 | 8,4 | 9,7 |

## Beispiel 58

Zu einer in der Wärme zubereiteten Lösung von 15,0 g (47,9 mmol) 1,4-Dihydro-4-imino-6-methoxy-1-phenylpyridazin-methylsulfat in 200 ml Wasser werden 100 ml Toluol gegeben. Das Zwei-Phasensystem wird bei Raumtemperatur unter Rühren zuerst mit 7,8 g (49,8 mmol) Chlorameisensäurephenylester und anschließend innerhalb 30 min mit einer Lösung von 4,0 g (100 mmol) Natriumhydroxid in 35 ml Wasser versetzt. Es wird 10 min bei Raumtemperatur nachgerührt. Anschließend werden die Phasen getrennt. Die rötliche Toluolphase wird getrocknet und eingeengt. Nach dem Umkristallisieren des Rückstandes aus Toluol unter Zusatz von Tierkohle erhält man 6,5 g (42% d. Th.) 1,4-Dihydro-6-methoxy-4-(phenoxycarbonyl)imino-1-phenylpyridazin, Schmelzpunkt 114°C.

Analyse für $C_{18}H_{15}N_3O_3$ (321,3):

| | | | |
|---|---|---|---|
| Ber. | C 67,3 | H 4,7 | N 13,1% |
| Gef. | C 67,2 | H 4,9 | N 13,1%. |

In der folgenden Tabelle (Tabelle 4) sind die Ausführungsbeispiele 59 bis 83 zusammengestellt. Die Herstellung dieser Pyridazinonimine erfolgt nach der im Beispiel 58 beschriebenen Methode. Als Substrat wird aber bei den Beispielen 61, 63, 65, 67, 70 und 75 anstatt 1,4-Dihydro-4-imino-6-methoxy-1-phenylpyridazin-methylsulfat 1,4-Dihydro-6-ethoxy-4-imino-1-phenylpyridazin-ethylsulfat eingesetzt. Weiterhin wird nur bei den Beispielen 69 bis 72 als Reagenz — wie beim Beispiel 58 — ein Chlorameisensäureester verwendet. Als Reagenz dient bei den übrigen Beispielen ein Carbonsäurechlorid (Beispiele 59 bis 68 und 76 bis 80), Chlorthioameisensäure-S-ethylester (Beispiel 73) bzw. ein Isocyanat (Beispiele 74, 75, 81, 82 und 83).

Bei Einsatz eines Isocyanats verwendet man auf 47,9 mmol Substrat nur 50 mmol Natriumhydroxid. Im Falle der Ausführungsbeispiele 62 bis 68, 72 bis 74 und 78 bis 83 wird nach der Zugabe der Natronlauge und dem Nachrühren bei Raumtemperatur abgesaugt. Die erhaltene Festsubstanz wird mit Wasser gewaschen, getrocknet und umkristallisiert.

Tabelle 4

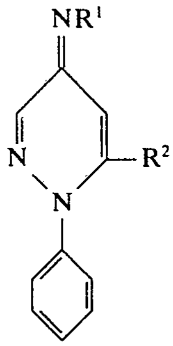

| Beispiel | $R^1$ | $R^2$ | Schmelzpunkt [°C] | Ausbeute (%) | Analyse (%) | C | H | N |
|---|---|---|---|---|---|---|---|---|
| 59 | $—CO—CH_2—$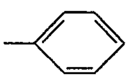 | $—OCH_3$ | 128–129 (Essigester) | 3 | Ber.<br>Gef. | 71,5<br>71,4 | 5,4<br>5,4 | 13,2<br>13,3 |
| 60 | —CO—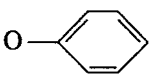 | $—OCH_3$ | 145–147 (Acetonitril) | 48 | Ber.<br>Gef. | 70,6<br>71,2 | 5,3<br>5,1 | 13,7<br>13,7 |
| 61 | —CO—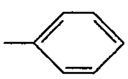 | $—OC_2H_5$ | 167 (Ethanol) | 69 | Ber.<br>Gef. | 71,5<br>71,4 | 5,4<br>5,5 | 13,2<br>13,4 |
| 62 | —CO—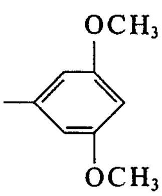 | $—OCH_3$ | 151 (Acetonitril) | 49 | Ber.<br>Gef. | 65,7<br>65,5 | 5,2<br>5,2 | 11,5<br>11,7 |
| 63 | —CO—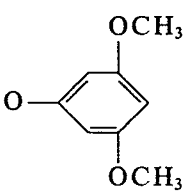 | $—OC_2H_5$ | 161 (Acetonitril) | 40 | Ber.<br>Gef. | 66,5<br>66,3 | 5,6<br>5,4 | 11,1<br>11,2 |

Tabelle 4 (Fortsetzung)

| Beispiel | $R^1$ | $R^2$ | Schmelzpunkt [°C] | Ausbeute (%) | Analyse (%) | C | H | Cl | N |
|---|---|---|---|---|---|---|---|---|---|
| 64 | —CO—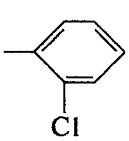 | $—OCH_3$ | 162 (Acetonitril) | 35 | Ber.<br>Gef. | 63,6<br>63,6 | 4,2<br>4,2 | 10,4<br>10,7 | 12,4<br>12,4 |
| 65 | —CO—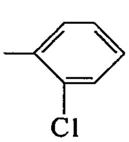 | $—OC_2H_5$ | 134 (Toluol) | 34 | Ber.<br>Gef. | 64,5<br>64,8 | 4,6<br>4,8 | 10,1<br>10,1 | 11,9<br>11,8 |

Tabelle 4 (Fortsetzung)

| Beispiel | $R^1$ | $R^2$ | Schmelzpunkt [°C] | Ausbeute (%) | Analyse (%) | C | H | Cl | N |
|---|---|---|---|---|---|---|---|---|---|
| 66 | —CO 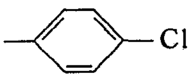 | $-OCH_3$ | 100 (Acetonitril) | 60 | Ber.<br>Gef. | 63,6<br>63,6 | 4,2<br>4,2 | 10,4<br>10,5 | 12,4<br>12,3 |
| 67 | —CO 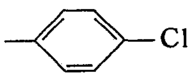 | $-OC_2H_5$ | 129 (Acetonitril) | 39 | Ber.<br>Gef. | 64,5<br>63,9 | 4,6<br>4,9 | 10,1<br>10,3 | 11,9<br>11,7 |
| 68 | —CO 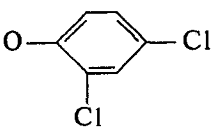 | $-OCH_3$ | 166 (Acetonitril) | 43 | Ber.<br>Gef. | 57,8<br>57,9 | 3,5<br>3,6 | 19,0<br>18,8 | 11,2<br>11,2 |
| 69 | $-CO-O-CH_2-CH_3$ | $-OCH_3$ | 110 (Ligroin) | 40 | Ber.<br>Gef. | 61,5<br>61,6 | 5,5<br>5,5 | -<br>- | 15,4<br>15,5 |

Tabelle 4 (Fortsetzung)

| Beispiel | $R^1$ | $R^2$ | Schmelzpunkt [°C] | Ausbeute (%) | Analyse (%) | C | H | N | S |
|---|---|---|---|---|---|---|---|---|---|
| 70 | $-CO-O-CH_2-CH_3$ | $-OC_2H_5$ | 70-72 (Cyclohexan) | 45 | Ber.<br>Gef. | 62,7<br>62,0 | 6,0<br>6,0 | 14,6<br>14,6 | -<br>- |
| 71 | $-CO-O-(CH_2)_3-CH_3$ | $-OCH_3$ | 115 (Toluol) | 47 | Ber.<br>Gef. | 63,8<br>63,8 | 6,4<br>6,4 | 13,9<br>14,0 | -<br>- |
| 72 | $-CO-O-CH_2-CH=CH_2$ | $-OCH_3$ | 115-120 (Toluol) | 28 | Ber.<br>Gef. | 63,2<br>62,7 | 5,2<br>5,3 | 14,7<br>14,4 | -<br>- |
| 73 | $-CO-S-CH_2-CH_3$ | $-OCH_3$ | 122 (Toluol) | 28 | Ber.<br>Gef. | 58,1<br>58,2 | 5,1<br>5,3 | 14,5<br>14,8 | 11,1<br>10,8 |
| 74 | $-CO-NH-CH_2-$ 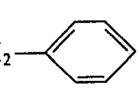 | $-OCH_3$ | 110 (Toluol) | 42 | Ber.<br>Gef. | 68,3<br>67,5 | 5,4<br>5,5 | 16,8<br>16,8 | -<br>- |
| 75 | $-CO-NH-$ 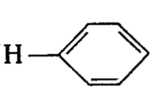 | $-OC_2H_5$ | 154 (Acetonitril) | 24 | Ber.<br>Gef. | 68,2<br>68,2 | 5,4<br>5,2 | 16,8<br>16,7 | -<br>- |

Tabelle 4 (Fortsetzung)

| Beispiel | $R^1$ | $R^2$ | Schmelzpunkt [°C] | Ausbeute (%) | Analyse (%) | C | H | Cl | F | N |
|---|---|---|---|---|---|---|---|---|---|---|
| 76 | —CO 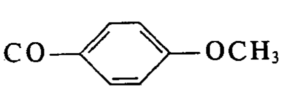 | $-OCH_3$ | 156-158 | 21 | Ber.<br>Gef. | 68,1<br>67,9 | 5,1<br>5,1 | -<br>- | -<br>- | 12,5<br>12,4 |

Fortsetzung

| Beispiel | R¹ | R² | Schmelzpunkt [°C] | Ausbeute (%) | Analyse (%) | C | H | Cl | F | N |
|---|---|---|---|---|---|---|---|---|---|---|
| 77 | —CO— 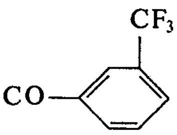 | $-OCH_3$ | 195-197 (Ethanol) Perchlorat | 44 | Ber. | 48,2 | 3,2 | 7,5 | 12,0 | 8,9 |
| | | | | | Gef. | 48,7 | 3,2 | 7,5 | 12,1 | 9,0 |
| 78 | —CO— 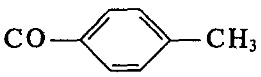 | $-OCH_3$ | 183-185 (Acetonitril) | 48 | Ber. | 71,5 | 5,4 | - | - | 13,2 |
| | | | | | Gef. | 71,3 | 5,3 | - | - | 13,3 |
| 79 | —CO— 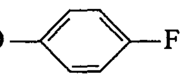 | $-OCH_3$ | 175-176 (Acetonitril) | 32 | Ber. | 66,9 | 4,4 | - | 5,9 | 13,0 |
| | | | | | Gef. | 66,7 | 4,5 | - | 5,9 | 13,2 |
| 80 | —CO— 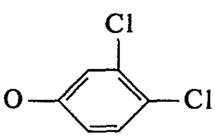 | $-OCH_3$ | 188-190 (Acetonitril) | 65 | Ber. | 57,8 | 3,5 | 19,0 | - | 11,2 |
| | | | | | Gef. | 57,3 | 3,7 | 18,5 | - | 11,2 |
| 81 | —CO—NH— 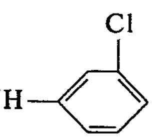 | $-OCH_3$ | 147-148 (Acetonitril) | 26 | Ber. | 60,9 | 4,3 | 10,0 | - | 15,8 |
| | | | | | Gef. | 61,2 | 4,4 | 9,9 | - | 15,8 |
| 82 | —CO—NH— 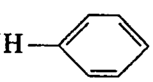 | $-OCH_3$ | 137-138 (Acetonitril) | 17 | Ber. | 67,5 | 5,0 | - | - | 17,5 |
| | | | | | Gef. | 67,5 | 5,1 | - | - | 17,5 |
| 83 | —CO—NH— 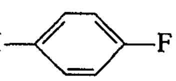 | $-OCH_3$ | 152-153 (Acetonitril) | 35 | Ber. | 63,9 | 4,5 | - | 5,6 | 16,6 |
| | | | | | Gef. | 64,0 | 4,5 | - | 5,7 | 16,5 |

## Beispiel 84

Zu der Lösung von 7,5 g (36,1 mmol) 1,4-Dihydro-4-imino-1-phenylpyridazin-hydrochlorid in 70 ml Wasser werden 70 ml Toluol gegeben. Das Zwei-Phasensystem wird bei Raumtemperatur unter Rühren zuerst mit 2,6 g (36,6 mmol) Ethylisocyanat und anschließend innerhalb 15 min mit 1,5 g (37,5 mmol) NaOH in 20 ml Wasser versetzt. Es wird noch 1 h bei Raumtemperatur gerührt und dann abgesaugt. Die erhaltene Festsubstanz wird mit Wasser gewaschen, getrocknet und zweimal aus Methylenchlorid/Hexan umkristallisiert. Man isoliert 4,0 g (46%d. Th.) 1,4-Dihydro-4-(ethylcarbamoyl)imino-1-phenylpyridazin, hellgelbe Kristalle, Schmelzpunkt 142 bis 143°C.

Analyse für $C_{13}H_{14}N_4O$ (242,3):

Ber. C 64,4 H 5,8 N 23,1%
Gef. C 64,3 H 5,9 N 23,3%

## Beispiel 85

Führt man das Beispiel 84 unter Verwendung von Propylisocyanat (3,1 g (36,4 mmol)) an Stelle von Ethylisocyanat durch, so erhält man nach der Umkristallisation aus Methylenchlorid/Hexan 5,1 g (55% d. Th.) 1,4-Dihydro-1-phenyl-4-[(propylcarbamoyl)imino]pyridazin, gelbbeige Kristalle, Schmelzpunkt 156 bis 157°C.

Analyse für $C_{14}H_{16}N_4O$ (256,3):

Ber. C 65,5 H 6,3 N 21,9%
Gef. C 65,7 H 6,3 N 22,0%

Beispiel 86

Zu 10,0 g (48,2 mmol) 1,4-Dihydro-4-imino-1-phenylpyridanzin-hydrochlorid in 25 ml (26,7 g (365 mmol)) Methylisothiocyanat werden bei Raumtemperatur unter Rühren 5,3 g (52,4 mmol) Triethylamin getropft. Nach beendeter Zugabe wird das Reaktionsgemisch mit 50 ml Aceton verdünnt und noch 2 h bei Raumtemperatur gerührt. Es wird abgesaugt, mit Wasser und Aceton nachgewaschen und aus Dimethylformamid/Wasser umkristallisiert. Die erhaltenen Kristalle schlämmt man in 25 ml Aceton auf, saugt ab, schlämmt nochmals in 25 ml Aceton auf, saugt ab und kristallisiert aus Isopropanol um. Man erhält 2,8 g (24% d. Th.) 1,4-Dihydro-4-(methylthiocarbamoyl)imino-1-phenylpyridazin, gelbe Kristalle, Schmelzpunkt 177 bis 178°C (Zersetzung).

Analyse für $C_{12}H_{12}N_4S$ (244,3):

| | | | |
|---|---|---|---|
| Ber. C 59,0 | H 5,0 | N 22,9 | S 13,1% |
| Gef. C 59,2 | H 4,9 | N 23,0 | S 12,8% |

Beispiel 87

Zu 10,0 g (48,2 mmol) 1,4-Dihydro-4-imino-1-phenylpyridazin-hydrochlorid in 25 ml (24,9 g (285 mmol)) Ethylisothiocyanat werden bei Raumtemperatur unter Rühren 5,3 g (52,4 mmol) Triethylamin getropft. Nach beendeter Zugabe wird das Reaktionsgemisch mit 50 ml Aceton verdünnt und noch 2 h bei Raumtemperatur gerührt. Nun wird filtriert. Der Filterrückstand wird verworfen. Das Filtrat engt man ein und kristallisiert den verbleibenden Feststoff zuerst aus Isopropanol und dann noch aus Essigester/Hexan um. Ausbeute: 1,0 g (8% d. Th.) 1,4-Dihydro-4-(ethylthiocarbamoyl)imino-1-phenylpyridazin, gelbe Kristalle, Schmelzpunkt 134 bis 135°C.

Analyse für $C_{13}H_{14}N_4S$ (258,3):

| | | | |
|---|---|---|---|
| Ber. C 60,4 | H 5,5 | N 21,7 | S 12,4% |
| Gef. C 60,1 | H 5,3 | N 21,8 | S 12,3% |

Beispiel 88

Zu 7,5 g (36,1 mmol) 1,4-Dihydro-4-imino-1-phenylpyridazin-hydrochlorid und 5,0 g (36,6 mmol) Oxalsäureethylesterchlorid in 100 ml Methylenchlorid werden bei Raumtemperatur unter Rühren 7,5 g (74,1 mmol) Triethylamin getropft. Nach beendeter Zugabe wird noch 1 h bei Raumtemperatur nachgerührt. Nun wird das Reaktionsgemisch mit Wasser versetzt. Die organische Phase wird abgetrennt, mit Wasser gewaschen und eingeengt. Den Rückstand kristallisiert man zuerst aus Essigester und dann noch aus Methylenchlorid/Petrolether um. Ausbeute: 3,9 g (40% d. Th.) 1,4-Dihydro-4-(ethoxyoxalyl)imino-1-phenylpyridazin, gelbe Kristalle, Schmelzpunkt 142 bis 143°C.

Analyse für $C_{14}H_{13}N_3O_3$ (271,3):

| | | |
|---|---|---|
| Ber. C 62,0 | H 4,8 | N 15,5% |
| Gef. C 61,9 | H 4,9 | N 15,6% |

Formulierungsbeispiele, die in üblicher Weise hergestellt werden:

1. Tabletten:

| | |
|---|---|
| Wirkstoff | 10 mg |
| Polyvinylpyrrolidon (mittl. M. G. 25 000) | 170 mg |
| Polyethylenglykol (mittl. M. G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyethylenglykol (mittl. M. G. 4 000), Hydroxypropylmethylcellulose, Talkum und Magnesiumstearat vermischt und zu Tabletten à 240 mg verpreßt.

2. Beispiel für Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. 1-Phenyl-4(1H)-pyridazinonimine der Formel (I),

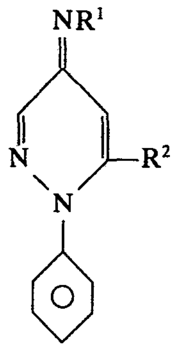

(I)

in der $R^1$ eine der folgenden Bedeutungen annehmen kann:

- einen Acylrest $-CO-R^3$, in dem $R^3$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, einen Cycloalkylrest mit 3 bis 8 C-Atomen im Ring, der gegebenenfalls ein- bis vierfach durch Alkylreste mit 1 bis 4 C-Atomen substituiert ist, einen Alkenylrest mit 2 bis 8 C-Atomen oder einen Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, steht,
- einen Rest $-CO-Y-R^4$, in dem Y für ein Sauerstoffatom oder ein Schwefelatom und $R^4$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, einen Alkenylrest mit 3 bis 8 C-Atomen, in dem die Doppelbindung durch eine Alkylenkette mit mindestens 1 C-Atom von Y getrennt ist, oder einen Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, stehen,
- einen Rest

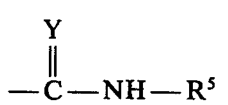

in dem Y für ein Sauerstoff- oder Schwefelatom und $R^5$ für einen Alkylrest mit 1 bis 8 C-Atomen, einen Benzylrest, der im Phenylring ein- bis dreifach durch gleiche oder verschiedene Substituenten aus der Gruppe Alkyl mit 1 bis 3 C-Atomen, Alkoxy mit 1 bis 3 C-Atomen im Alkyl, Halogen oder Trifluormethyl substituiert sein kann, oder eine Phenylrest, der gegebenenfalls ein- bis dreifach durch gleiche oder verschiedene Substituenten aus der Gruppe Alkyl mit 1 bis 4 C-Atomen, Alkoxy mit 1 bis 4 C-Atomen im Alkyl, Halogen oder Trifluormethyl substituiert ist, stehen, oder

– einen Rest $-CO-COO-R^6$, in dem $R^6$ für einen Alkylrest mit 1 bis 8 C-Atomen steht; und

$R^2$ ein Wasserstoffatom oder einen Alkoxyrest mit 1 bis 8 C-Atomen im Alkyl bedeutet; sowie ihre Säureadditionssalze mit einer physiologisch verträglichen Säure.

2. Verbindungen der Formel (I) nach Anspruch 1, in der $R^1$ eine der folgenden Bedeutungen annehmen kann:

– einen Acylrest $-CO-R^3$, in dem $R^3$ für einen Alkylrest mit 1 bis 8 C-Atomen, einen Benzylrest, der im Phenylring einfach durch Fluor, Chlor oder Methoxy substituiert sein kann, einen Cycloalkylrest mit 3 bis 6 C-Atomen im Ring, der gegebenenfalls ein- oder zweifach durch eine Methylgruppe substituiert ist, einen Alkenylrest mit 2 bis 4 C-Atomen oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, steht,

– einen Rest $-CO-Y-R^4$, in dem Y für ein Sauerstoffatom oder ein Schwefelatom und $R^4$ für einen Alkylrest mit 1 bis 4 C-Atomen, einen Benzylrest, der im Phenylring einfach durch Fluor, Chlor oder Methoxy substituiert sein kann, einen Alkenylrest mit 3 bis 6 C-Atomen, in dem die Doppelbindung durch eine Alkylenkette mit mindestens 1 C-Atom von Y getrennt ist, oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, stehen,

– einen Rest

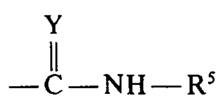

in dem Y für ein Sauerstoff- oder Schwefelatom und $R^5$ für einen Alkylrest mit 1 bis 6 C-Atomen, einen Benzylrest, der im Phenylring einfach durch Fluor, Chlor oder Methoxy substituiert sein kann, oder einen Phenylrest, der gegebenenfalls ein- oder zweifach durch einen Substituenten aus der Gruppe Alkyl mit 1 bis 3 C-Atomen, Alkoxy mit 1 bis 3 C-Atomen im Alkyl, Halogen oder Trifluormethyl substituiert ist, stehen, oder

– einen Rest $-CO-COO-R^6$, in dem $R^6$ für einen Alkylrest mit 1 bis 4 C-Atomen steht; und

$R^2$ ein Wasserstoffatom oder einen Alkoxyrest mit 1 bis 4 C-Atomen im Alkyl bedeuten; sowie ihre Säureadditionssalze mit einer physiologisch verträglichen Säure.

3. 1,4-Dihydro-1-phenyl-4-[(propylcarbamoyl)-imino]pyridazin und seine Säureadditionssalze nach Anspruch 1.

4. 1,4-Dihydro-4-(methylcarbamoyl)imino-1-phenylpyridazin und seine Säureadditionssalze nach Anspruch 1.

5. 1,4-Dihydro-4-propionylimino-6-methoxy-1-phenylpyridazin und seine Säureadditionssalze nach Anspruch 1.

6. 1,4-Dihydro-4-methacryloylimino-1-phenylpyridazin und seine Säureadditionssalze nach Anspruch 1.

7. 1,4-Dihydro-4-(phenoxycarbonyl)imino-1-phenylpyridazin und seine Säureadditionssalze nach Anspruch 1.

8. Verfahren zur Herstellung von Verbindungen der Formel (I) nach Anspruch 1, worin $R^1$ der Acylrest $-CO-R^3$ ist, dadurch gekennzeichnet, daß man eine Verbindung der Formel (III)

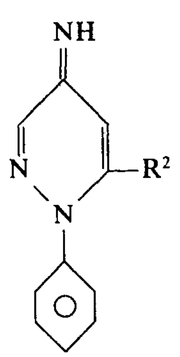

(III)

in der $R^2$ die in Anspruch 1 angegebenen Bedeutungen hat, gegebenenfalls in Form eines beliebigen Säureadditionssalzes, mit einem Acylierungsmittel der Formel (IV)

$$R^3—CO—A \qquad (IV)$$

worin A für ein Halogenatom oder den Rest $—O—CO—R^3$ steht, und $R^3$ wie in Anspruch 1 olefiniert ist, behandelt und die jeweils erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

9. Verfahren zur Herstellung von Verbindungen der Formel (I) nach Anspruch 1, worin $R^1$ der Rest $—CO—Y—R^4$ ist, dadurch gekennzeichnet, daß man eine Verbindung der Formel (III)

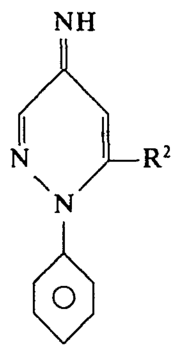

(III)

in der $R^2$ die in Anspruch 1 angegebenen Bedeutungen hat, gegebenenfalls in Form eines beliebigen Säureadditionssalzes mit einer Verbindung der Formel (V)

$$R^4—Y—COB \qquad (V)$$

worin B für ein Halogenatom steht, und Y und $R^4$ wie in Anspruch 1 definiert sind, miteinander umsetzt und die jeweils erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

10. Verfahren zur Herstellung von Verbindungen der Formel (I) nach Anspruch 1, worin $R^1$ der Rest

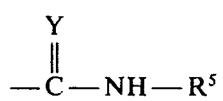

ist, dadurch gekennzeichnet, daß man eine Verbindung der Formel (III)

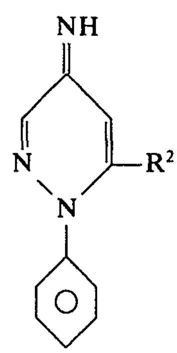

(III)

in der $R^2$ die in Anspruch 1 angegebenen Bedeutungen hat, gegebenenfalls in Form eines beliebigen Säureadditionssalzes mit einer Verbindung der Formel (VI)

$$R^5—N{=}C{=}Y \qquad (VI)$$

worin $R^5$ und Y wie in Anspruch 1 definiert sind, miteinander umsetzt und die jeweils erhaltene Verbindung, wenn es sich um das freie Imin handelt, gegebenenfalls in bekannter Weise in das Säureadditionssalz einer physiologisch verträglichen Säure überfüht, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

11. Verfahren zur Herstellung von Verbindungen der Formel (I) nach Anspruch 1, worin $R^1$ der Rest $-CO-COO-R^6$ bedeutet, dadurch gekennzeichnet, daß man eine Verbindung der Formel (III)

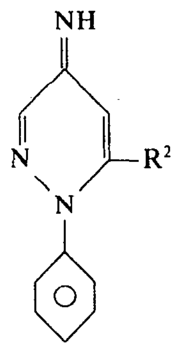

(III)

in der $R^2$ die in Anspruch 1 angegebenen Bedeutungen hat, gegebenenfalls in Form eines beliebigen Säureadditionssalzes mit einem Oxalsäureesterchlorid der Formel (VII)

$$R^6-OOC-CO-Cl \quad (VII)$$

worin $R^6$ wie in Anspruch 1 definiert ist, miteinander umsetzt und die jeweils erhaltene Verbindung, wenn es sich um das freie Imin handelt, gegebenenfalls in bekannter Weise in das Säureadditionssalz einer physiologisch verträglichen Säure überführt, oder, wenn ein Säureadditionssalz erhalten wird, dieses gegebenenfalls in bekannter Weise in das Säureadditionssalz einer anderen, und zwar physiologisch verträglichen, Säure oder in das freie Imin überführt.

12. Verfahren nach Anspruch 8, dadurch gekennzeichnet, daß $R^2$ und $R^3$ wie in Anspruch 2 definiert sind.

13. Verfahren nach Anspruch 9, dadurch gekennzeichnet, daß $R^2$, $R^4$ und Y wie in Anspruch 2 definiert sind.

14. Verfahren nach Anspruch 10, dadurch gekennzeichnet, daß $R^2$, $R^5$ und Y wie in Anspruch 2 definiert sind.

15. Verfahren nach Anspruch 11, dadurch gekennzeichnet, daß $R^2$ und $R^6$ wie in Anspruch 2 definiert sind.

16. Therapeutische Mittel, enthaltend eine Verbindung der Formel (I) nach Anspruch 1 oder eines ihres physiologisch verträglichen Säureadditionssalze als Wirkstoff neben pharmazeutisch üblichen Träger- und Verdünnungsmitteln und pharmazeutisch-technischen Hilfsstoffen.

**Claims**

1. A 1-phenyl-4(1H)-pyridazinone-imine of the formula (I)

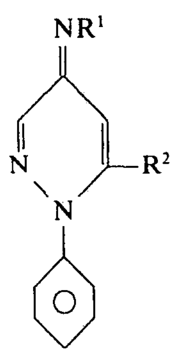

(I)

where $R^1$ can have one of the following meanings:

- an acyl radical $-CO-R^3$, where $R^3$ is alkyl of 1 to 8 carbon atoms or benzyl which can be monosubstituted, disubstituted or trisubstituted in the phenyl ring by identical or different substituents from the group comprising alkyl of 1 to 3 carbon atoms, alkoxy where alkyl is of 1 to 3 carbon atoms, halogen and trifluoromethyl, or ist cycloalkyl of 3 to 8 ring carbon atoms which is unsubstituted or monosubstituted, disubstituted, trisubstituted or tetrasubstituted by alkyl of 1 to 4 carbon atoms, or is alkenyl or 2 to 8 carbon atoms, or phenyl which is unsubstituted or monosubstituted, disubstituted or trisubstituted by identical or different substituents from the group comprising alkyl of 1 to

4 carbon atoms, alkoxy where alkyl is of 1 to 4 carbon atoms, halogen and trifluoromethyl,

– $-CO-Y-R^4$, where Y is oxygen or sulfur, and $R^4$ is alkyl of 1 to 8 carbon atoms, or benzyl which can be monosubstituted, disubstituted or trisubstituted in the phenyl ring by identical or different substituents from the group comprising alkyl of 1 to 3 carbon atoms, alkoxy where alkyl is of 1 to 3 carbon atoms, halogen and trifluoromethyl, or is alkenyl which is of 3 to 8 carbon atoms and in which the double bond is separated from Y by an alkylene chain of one or more carbon atoms, or is phenyl which is unsubstituted or monosubstituted, disubstituted or trisubstituted by identical or different substituents from the group comprising alkyl of 1 to 4 carbon atoms, alkoxy where alkyl is of 1 to 4 carbon atoms, halogen and trifluoromethyl,

– 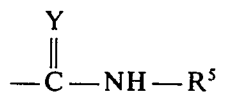

where Y is oxygen or sulfur, and $R^5$ is alkyl of 1 to 8 carbon atoms, or benzyl which can be monosubstituted, disubstituted or trisubstituted in the phenyl ring by identical or different substituents from the group comprising alkyl of 1 to 3 carbon atoms, alkoxy where alkyl is of 1 to 3 carbon atoms, halogen and trifluoromethyl, or is phenyl which is unsubstituted or monosubstituted, disubstituted or trisubstituted by identical or different substituents from the group comprising alkyl of 1 to 4 carbon atoms, alkoxy where alkyl is of 1 to 4 carbon atoms, halogen and trifluoromethyl, or

– $-CO-COO-R^6$, where $R^6$ is alkyl of 1 to 8 carbon atoms, and

$R^2$ is hydrogen or alkoxy where alkyl is of 1 to 8 carbon atoms,

and its addition salts with a physiologically tolerated acid.

2. A compound of the formula (I) as claimed in claim 1, where $R^1$ can have one of the following meanings:

– an acyl radical $-CO-R^3$, where $R^3$ is alkyl of 1 to 8 carbon atoms, or benzyl which can be monosubstituted in the phenyl ring by fluorine, chlorine or methoxy, or is cycloalkyl of 3 to 6 ring carbon atoms which is unsubstituted or monosubstituted or disubstituted by methyl, or is alkenyl of 2 to 4 carbon atoms, or phenyl which is unsubstituted or monosubstituted or disubstituted by a substituent from the group comprising alkyl of 1 to 3 carbon atoms, alkoxy where alkyl is of 1 to 3 carbon atoms, halogen and trifluoromethyl,

– $-CO-Y-R^4$ where Y is oxygen or sulfur, and $R^4$ is alkyl or 1 to 4 carbon atoms or benzyl which can be monosubstituted in the phenyl ring by fluorine, chlorine or methoxy, or is alkenyl which is of 3 to 6 carbon atoms an in which the double bond is separated from Y by an alkylene chain of one or more carbon atoms, or is phenyl which is unsubstituted or monosubstituted or disubstituted by a substituent from the group comprising alkyl of 1 to 3 carbon atoms, alkoxy where alkyl is of 1 to 3 carbon atoms, halogen and trifluoromethyl,

– 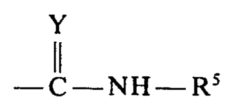

where Y is oxygen or sulfur, and $R^5$ is alkyl of 1 to 6 carbon atoms, or benzyl which can be monosubstituted in the phenyl ring by fluorine, chlorine or methoxy, or is phenyl which is unsubstituted or monosubstituted or disubstituted by a substituent from the group comprising alkyl of 1 to 3 carbon atoms, alkoxy where alkyl is of 1 to 3 carbon atoms, halogen and trifluoromethyl, or

– $-CO-COO-R^6$ where $R^6$ is alkyl of 1 to 4 °C , and

$R^2$ is hydrogen or alkoxy where alkyl is of 1 to 4 carbon atoms,

and its additions salts with a physiologically tolerated acid.

3. 1,4-Dihydro-1-phenyl-4-[(propylcarbamyl)-imino]-pyridazine, and its addition salts with an acid, as claimed in claim 1.

4. 1,4-Dihydro-4-(methylcarbamyl)-imino-1-phenylpyridazine, and its addition salts with an acid, as claimed in claim 1.

5. 1,4-Dihydro-4-propionylimino-6-methoxy-1-phenylpyridazine, and its addition salts with an acid, as claimed in claim 1.

6. 1,4-Dihydro-4-methacryloylimino-1-phenylpyridazine and its addition salts with an acid, as claimed in claim 1.

7. 1,4-Dihydro-4-(phenoxycarbonyl)-imino-1-phenylpyridazine, and its addition salts with an acid, as claimed in claim 1.

8. A process for the preparation of a compound of the formula (I) as claimed in claim 1, where $R^1$ is the acyl radical $-CO-R^3$, wherein a compound of the formula (III)

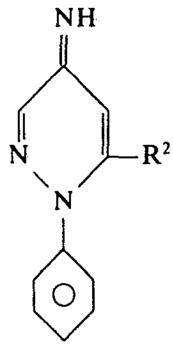

(III)

where $R^2$ has the meanings given in claim 1, if appropriate in the form of any desired addition salt an acid, is treated with an acylating agent of the formula (IV)

$$R^3—CO—A \quad (IV)$$

where A is halogen or $-O-CO-R^3$, and $R^3$ is as defined in claim 1, and if the compound obtained in each case is the free imine, this may, if required, be converted in a conventional manner into an addition salt with a physiologically tolerated acid, or if an addition salt with an acid is obtained, this salt may, if required, be converted in a conventional manner into an addition salt with another, physiologically tolerated acid, or into the free imine.

9. A process for the preparation of a compound of the formula (I) as claimed in claim 1, where $R^1$ is $-COY-R^4$, wherein a compound of the formula (III)

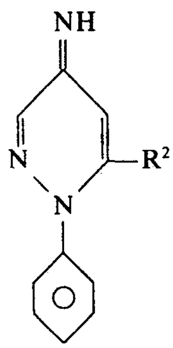

(III)

where $R^2$ has the meanings given in claim 1, if appropriate in the form of any desired addition salt with an acid, is reacted with a compound of the formula (V)

$$R^4—Y—COB \quad (V)$$

where B is halogen, and Y and $R^4$ are as defined in claim 1, and if the compound obtained in each case is free imine, this may, if required, be converted in a conventional manner into an addition salt with a physiologically tolerated acid, or if an addition salt with an acid is obtained, this salt may, if required, be converted in a conventional manner into an addition salt with another, physiologically tolerated acid, or into the free imine.

10. A process for the preparation of a compound of the formula (I) as claimed in claim 1, where $R^1$ is

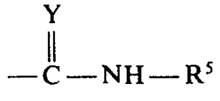

wherein a compound of the formula (III)

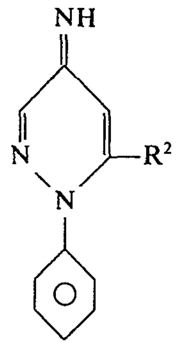

(III)

where $R^2$ has the meanings given in claim 1, if appropriate in the form of any desired addition salt with an acid, is reacted with a compound of the formula (VI)

$$R^5—N=C=Y \quad (VI)$$

where $R^5$ and Y are as defined in claim 1, and if the compound obtained in each case is the free imine, this may, if required, be converted in a conventional manner into an addition salt with a physiologically tolerated acid, or if an addition salt with an acid is obtained, this salt may, if required, be converted in a conventional manner into an addition salt with another, physiologically tolerated acid, or into the free imine.

11. A process for the preparation of a compound of the formula (I) as claimed in claim 1, where $R^1$ is $-CO-COO-R^6$, wherein a compound of the formula (III)

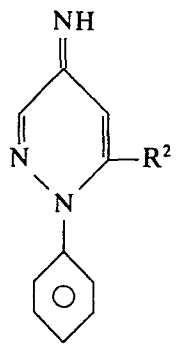

(III)

where $R^2$ has the meanings given in claim 1, if appropriate in the form of any desired addition salt with an acid, is reacted with an oxalic acid ester chloride of the formula (VII)

$$R^6—OOC—CO—Cl \quad (VII)$$

where $R^6$ is as defined in claim 1, and if the compound obtained in each case is the free imine, this may, if required, be converted in a conventional manner into an addition salt with a physiologically tolerated acid, or if an addition salt with an acid is obtained, this salt may, if required, be converted in a conventional manner into an addition salt with another, physiologically tolerated acid, or into the free imine.

12. A process as claimed in claim 8, wherein $R^2$ and $R^3$ are as defined in claim 2.

13. A process as claimed in claim 9, wherein $R^2$, $R^4$ and Y are as defined in claim 2.

14. A process as claimed in claim 10, wherein $R^2$, $R^5$ and Y are as defined in claim 2.

15. A process as claimed in claim 11, wherein $R^2$ and $R^6$ are as defined in claim 2.

16. A therapeutic agent which contains a compound of the formula (I) as claimed in claim 1, or one of its addition salts with a physiologically tolerated acid, as the active compound, in addition to conventional pharmaceutical carriers and diluents and pharmaceutical auxiliaries.

**Revendications**

1. 1-phényl-4(1H)-pyridazinonimines de formule I,

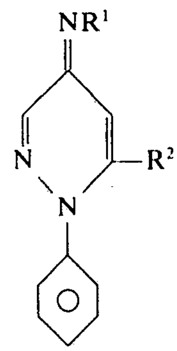

(I)

dans laquelle $R^1$ peut avoir une des significations suivantes:

– un reste acyle $-CO-R^3$, dans laquelle $R^3$ représente un reste alkyle ayant 1 à 8 atomes C, un reste benzyle qui peut être substitué dans le noyau phényle une à trois fois par des substituants identiques ou différents du groupe alkyle ayant 1 à 8 atomes C, alcoxy ayant 1 à 3 atomes C dans l'alkyle, halogène ou trifluorométhyle, un reste cycloalkyle de 3 à 8 atomes C dans le noyau, qui est éventuellement substitué de une à quatre fois par des restes alkyle ayant 1 à 4 atomes C, un reste alcényle de 2 à 8 atomes C ou un reste phényle, qui est éventuellement substitué une à trois fois par des substituants identiques ou différents du groupe alkyle de 1 à 4 atomes, alcoxy de 1 à 4 atomes C dans l'alkyle, halogène ou trifluorméthyle,
– un reste $-CO-Y-R^4$, dans lequel Y représente un atome d'oxygène ou un atome de soufre et $R^4$ un reste alkyle de 1 à 8 atomes C, un reste benzyle qui peut être substitué une à trois par des substituants identiques ou différents du groupe alkyle de 1 à 3 atomes C, alcoxy de 1 à 3 atomes C dans l'alkyle, halogène ou trifluorométhyle, un reste alcényle de 3 à 8 atomes C, dans lequel la double liaison est séparée de Y par une chaîne alkylène d'au moins un atome C, ou un reste phényle qui est éventuellement substitué une à trois par des substituants identiques ou différents du groupe alkyle de 1 à 4 atomes C, alcoxy de 1 à 4 atomes C dans l'alkyle, halogène ou trifluorméthyle,
– un reste

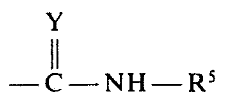

dans lequel Y représente un atome d'oxygène ou de soufre et $R^5$ un reste alkyle de 1 à 8 atomes C, un reste benzyle qui peut être substitué dans le noyau phényle, une à trois fois par des substituants identiques ou différents du groupe alkyle de 1 à 3 atomes C, alcoxy de 1 à 3 atomes C dans l'alkyle, halogène ou trifluorméthyle, ou un reste phényle qui est éventuellement substitué une à trois fois par des substituants identiques ou différents du groupe alkyle de 1 à 4 atomes C, alcoxy de 1 à 4 atomes C dans l'alkyle, halogène ou trifluorométhyle, ou
– un reste $-CO-COO-R^6$, dans lequel $R^6$ représente un reste alkyle de 1 à 8 atomes C, et
$R^2$ représente un atome d'hydrogène ou un reste alcoxy ayant 1 à 8 atomes C dans l'alkyle

ainsi que leurs sels d'addition avec un acide acceptable physiologiquement.

2. Composé de formule (I) selon la revendication 1, dans lequel $R^1$ peut prendre une des significations suivantes:

– un reste alcyle $-CO-R^3$, dans lequel $R^3$ représente un reste alkyle de 1 à 8 atomes C, un reste benzyle, qui peut être substitué dans le noyau phényle une fois par du fluore, chlore ou une méthoxy, un reste cycloalkyle de 3 à 6 atomes C dans le noyau, qui est éventuellement substitué une à deux fois par un groupe méthyle, un reste alcényle de 2 à 4 atomes C ou un reste phényle, qui est éventuellement substitué une ou deux fois par un substituant du groupe alkyle de 1 à 3 atomes C, alcoxy de 1 à 3 atomes C dans l'alkyle, halogène ou trifluorométhyle,
– un reste $-CO-Y-R^4$, dans lequel Y représente un atome d'oxygène ou un atome soufre et $R^4$ un reste alkyle de 1 à 4 atomes C, un reste benzyle, qui peut être substitué une fois par fluor, chlore ou méthoxy, un reste alcényle de 3 à 6 atomes C, dans lequel la double liaison est séparée de Y par une chaîne alkylène ayant au moins un atome C, ou un reste phényle qui est éventuellement substitué une ou deux fois par un substituant du groupe alkyle de 1 à 3 atomes C, alcoxy de 1 à 3 atomes C dans l'alkyle, halogène ou trifluorométhyle,
– un reste

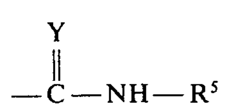

dans lequel Y représente un atome d'oxygène ou de soufre et $R^5$ un reste alkyle de 1 à 6 atomes C, un reste benzyle qui peut être substitué dans le noyau phényle une fois par du fluor, chlore ou méthoxy, ou un reste phényle qui est substitué éventuellement une fois ou deux par un substituant du groupe alkyle de 1 à 3 atomes C, alcoxy de 1 à 3 atomes C dans l'alkyle, halogène ou trifluorométhyle, ou

– un reste $-CO-COO-R^6$, dans lequel $R^6$ représente un reste alkyle de 1 à 4 atomes C, et

$R^2$ un atome d'hydrogène ou un reste alcoxy de 1 à 4 atomes C dans l'alkyle

ainsi que leurs sels d'addition d'un acide acceptable physiologiquement.

3. 1,4-dihydro-1-phényle-4-[(propylcarbamoyl)-imino]pyridazine et son sel d'addition d'acide selon la revendication 1.

4. 1,4-dihydro-4-(méthylcarbamoyl)imino-1-phénylpyridazine et ses sels d'addition d'acide selon la revendication 1.

5. 1,4-dihydro-4-propionylimino-6-méthoxy-1-phénylpyridazine et ses sels d'addition d'acide selon la revendication 1.

6. 1,4-dihydro-4-méthacryloylimino-1-phénylpyridazine et ses sels d'addition d'acide selon la revendication 1.

7. 1,4-dihydro-4-(phénoxycarbonyl)imino-1-phénylpyridazine et ses sels d'addition d'acide selon la revendication 1.

8. Procédé de préparation de composés de formule (I) selon la revendication 1 où $R^1$ est le reste alcyle $-CO-R^3$, caractérisé le fait que l'on traite un composé de formule (III)

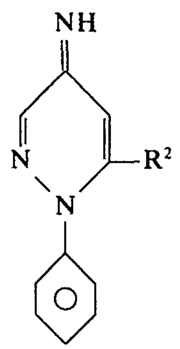

(III)

dans laquelle $R^2$ a les significations données dans la revendication 1, éventuellement sous forme d'un sel d'addition d'acide quelconque avec un agent d'acylation de formule (IV)

$$R^3-CO-A \qquad (IV)$$

dans laquelle A représente un atome d'halogène ou le reste $-O-CO-R^3$ et $R^3$ est défini comme dans la revendication 1, et l'on transforme le composé obtenu, lorsqu'il s'agit de l'imine libre, éventuellement, de manière connue, dans le sel d'addition d'un acide acceptable physiologiquement, ou, si l'on a obtenu un sel d'addition d'acide, celui-ci est transformé éventuellement de manière connue, dans le sel d'addition d'un autre acide, et acceptable physiologiquement ou dans l'imine libre.

9. Procédé pour la préparation de composés de formule (I) selon la revendication 1, dans lequel $R^1$ est le reste $COY-R^4$, caractérisé par le fait que l'on fait réagir l'un avec l'autre un composé de formule (III)

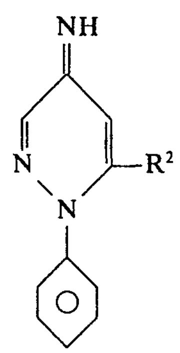

(III)

dans laquelle $R^2$ a les significations données dans la revendication 1, éventuellement sous forme d'un sel d'addition d'acide arbitraire avec un composé de formule (V)

$$R^4-Y-COB \qquad (V)$$

où B représente un atome d'halogène, et Y et $R^4$ sont définis comme dans la revendication 1, et l'on transforme le composé obtenu, lorsqu'il s'agit de l'imine libre, éventuellement, de manière connue, dans le sel d'addition d'un acide acceptable physiologiquement, ou, quand on a obtenu un sel d'addition d'acide, on transforme celui-ci éventuellement, de manière connue, dans le sel d'addition d'un autre acide acceptable physiologiquement ou bien dans l'imine libre.

10. Procédé pour la préparation de composés de formule (I) selon la revendication 1, dans lequel $R^1$ est le reste

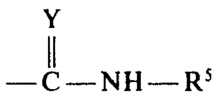

caractérisé par le fait que l'on fait réagir l'un avec l'autre un composé de formule (III)

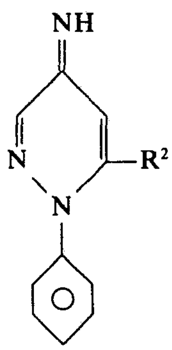

(III)

dans laquelle $R^2$ a les significations indiquées dans la revendication 1, éventuellement sous forme d'un sel d'addition d'acide voulu avec un composé de formule (VI)

$$R^5—N=C=Y \qquad (VI)$$

où $R^5$ et Y sont définis comme dans la revendication 1, et l'on transforme le composé obtenu, lorsqu'il s'agit de l'imine libre, éventuellement, de manière connue, dans le sel d'addition d'un acide acceptable physiologiquement, ou bien lorsqu'on a obtenu un sel d'addition d'acide, celui-ci est transformé éventuellement, de manière connue, dans le sel d'addition d'un autre acide, à savoir un acide acceptable physiologiquement ou bien dans l'imine libre.

11. Procédé pour la préparation de composés de formule (I) selon la revendication 1, dans lequel $R^1$ est le reste $-CO-COO-R^6$, caractérisé par le fait que l'on fait réagir l'un avec l'autre un composé de formule (III)

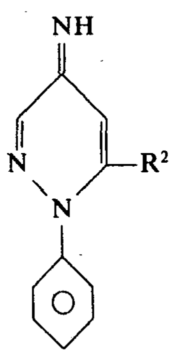

(III)

dans laquelle $R^2$ a la signification indiquée dans la revendication 1, éventuellement sous forme d'un sel d'addition d'acide arbitraire avec un chlorure d'ester d'acide oxalique de formule (VII)

$$R^6—OOC—CO—Cl \qquad (VII)$$

dans laquelle $R^6$ est défini comme dans la revendication 1, et l'on transforme le composé ainsi obtenu, lorsqu'il s'agit de l'imine libre, éventuellement, de manière connue, dans un sel d'addition d'acide d'un acide acceptable physiologiquement, ou bien lorsqu'on a obtenu un sel d'addition d'acide, celui-ci est transformé éventuellement, de manière connue, dans le sel d'addition d'un autre acide acceptable physiologiquement, ou bien dans l'imine libre.

12. Procédé selon la revendication 8, caractérisé par le fait que $R^2$ et $R^3$ sont définis comme dans la

revendication 2.

13. Procédé selon la revendication 9, caractérisé par le fait que $R^4$ et Y sont définis comme dans la revendication 2.

14. Procédé selon la revendication 10, caractérisé par le fait que, $R^2$, $R^5$ et Y sont définis comme dans la revendication 2.

15. Procédé selon la revendication 11, caractérisé par le fait que $R^2$ et $R^6$ sont définis comme dans la revendication 2.

16. Agent thérapeutique contenant comme principe actif, à côté d'agents supports et diluants usuels du point de vue pharmaceutique et d'auxiliaires techniques du point de vue pharmaceutique également, un composé de formule (I) selon la revendication 1 où un de ses sels d'addition d'un acide acceptable physiologiquement.